(19) Europäisches Patentamt
European Patent Office
Office européen des brevets

(11) **EP 4 254 423 A1**

(12) **EUROPEAN PATENT APPLICATION**

(43) Date of publication:
**04.10.2023 Bulletin 2023/40**

(21) Application number: **23164522.7**

(22) Date of filing: **28.03.2023**

(51) International Patent Classification (IPC):
***G16H 20/17*** *(2018.01)*

(52) Cooperative Patent Classification (CPC):
**G16H 20/17**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **30.03.2022 US 202263325362 P**

(71) Applicant: **Insulet Corporation**
**Acton MA 01720 (US)**

(72) Inventors:
• **LEE, Joon Bok**
**Acton, 01720 (US)**

• **BENJAMIN, Eric**
**Cambridge, 02138 (US)**
• **O'CONNOR, Jason**
**Acton, 01720 (US)**
• **ZHENG, Yibin**
**Hartland, 53029 (US)**
• **ZADE, Ashutosh**
**San Diego, 92128 (US)**

(74) Representative: **Peterreins Schley**
**Patent- und Rechtsanwälte PartG mbB**
**Hermann-Sack-Straße 3**
**80331 München (DE)**

(54) **AUTONOMOUS ADAPTIVITY AND SET POINT CONTROL FOR A DRUG DELIVERY DEVICE**

(57) Disclosed are techniques, processes, systems, and programming code that enables users of automatic drug delivery devices to initiate the automatic drug delivery device for an initial use phase as well as implement changes that control the adjustment of target setpoints by a medication delivery algorithm used in the control of the automatic drug delivery device. During the initial use phase, the automatic drug delivery device may be based on the user's glucose control outcome without performing time consuming and frequent settings of sensor values as well as allowing users to remain substantially within range of any target setpoints. In addition, the disclosed techniques, processes, systems, and programming code enable users to have initial dosage settings automatically set without having to input what were previously considered fundamental inputs.

FIG. 1

EP 4 254 423 A1

**Description**

BACKGROUND

The capability to remain within range of a blood glucose setting target value is a critical part in the typical diabetes care regimen for the user of a wearable automatic insulin device (AID) system. However, over time setting the proper target may become a tiresome task for the user to have to perform.

In addition, current AID systems require a significant amount of user input to initialize and behave safely and reduce the risk of hypoglycemia. However, euglycemic and safe glycemic ranges are similar for all users, and AID algorithms are typically already designed to be robust against significant errors in the input parameters entered by users. Therefore, a robust and safe design of an AID system can deliver insulin safely for users without requiring users to provide inputs previously considered fundamental to these systems, such as a glucose control target or basal profile.

Maintaining a healthy lifestyle is both critically important to the Type -1 diabetes patients and challenging at the same time. Many patients have challenges in maintaining blood glucose level during or after exercise and may have to take rescue snacks if there is a concern of hyperglycemia. Additionally, patients may feel fearful of excessive exercising and may be limiting their ability as exercise increases insulin absorption and normal dose of insulin while not exercising may be higher than necessary. The aim of a diabetes management regimen is to keep the user's blood glucose within the euglycemic range. The euglycemic ranges are similar for all users. Typically, a user when initializing a wearable drug delivery device is required to enter a basal profile, which may be multiple basal rates that each cover a different particular time period (e.g., 6am - noon) until an entire 24 hour period of time has an assigned basal rate. This process, for example, when the user's days are not exactly the same may become challenging and may also become tiresome over time.

As a result of these issues, the wearable automatic drug delivery device may not operate in an optimal fashion. Hence, there is a need for further automation and more effective setting of parameters related to different aspects of a drug delivery regimen.

BRIEF SUMMARY

In a first aspect, a computer-implemented method is provided comprising determining a user is initiating a wearable drug delivery device for an initial use. In response to determining the initial use, a high analyte target value and a low analyte target value is set or obtained, an initial basal amount of a liquid drug is set or obtained, a set duration is started, and the wearable drug device is caused to begin delivery of the initial basal amount. Sensor analyte values are obtained at predetermined time intervals within the set duration. A respective sensor analyte value is evaluated against the high target analyte value and the low target analyte value. A high target count is increased for each obtained sensor analyte value being greater than the high target analyte value. A low target count is increased for each obtained sensor analyte value being less than the low target analyte value. The initial basal amount is modified at predetermined time increments within the set duration by an adjustment amount of liquid drug. For each predetermined time increment, the adjustment amount is determined based on the current high target count and the current low target count. In embodiments, the high target analyte value and/or the low target analyte value may be predetermined, for instance stored on a memory, provided by a controller and/or provided by a user or clinician.

In embodiments, the adjustment amount may be determined based on a set adjustment factor and a variable count factor. For each predetermined time increment, the variable count factor may be determined based on the current high target count and the current low target count.

In embodiments, the set adjustment factor may be a set amount of liquid drug selected from 0.005 to 0.4, specifically from 0.01 to 0.2, more specifically from 0.05 to 0.1. In examples equal to or at least 0.005 units of the liquid drug, specifically equal to or at least 0.05 units of the liquid drug.

In embodiments, the variable count factor may be a positive integer which is multiplied with the set adjustment factor to determine the adjustment amount. In some embodiments, the variable count factor may assume a positive integer or a negative integer. Specifically, in examples where a higher initial bolus amount is selected, the variable count factor may be defined to assume a negative integer.

In embodiments, the variable the variable count factor may be increased by one integer per each number of high target counts corresponding to the number of predetermined time intervals fitting within the predetermined time increment.

In embodiments, the variable count factor may always be decreased by one integer for each predetermined time increment. Additionally, the variable count factor may be increased by one integer per each number of low target counts corresponding to the number of predetermined time intervals fitting within the predetermined time increment.

In embodiments, determining the user is initiating the drug delivery device for the initial use may comprise receiving an input from a user input device indicating that the user is a new user. Additionally or alternatively, determining the user is initiating the drug delivery device for the initial use may comprise receiving a signal from a controller indicating that the user is a new user. Additionally or alternatively, determining the user is initiating the drug delivery device for the initial use may comprise establishing a wireless communication connection with a controller device and receiving a signal indicating the user is a new user.

In embodiments, the computer-implemented method may further comprise selecting the initial basal amount from a default basal amount. The basal amount may be a number of units of the liquid drug delivered per a time increment. The number of units may have a range of 0.5 to 4.0 units, specifically a range of 1.0 to 3.0 units. The time increment may have a range of 0.2 to 6.0 hours, specifically a range of 0.5 to 3.0 hours.

In embodiments, the set duration may be a period of time over which a number of the obtained sensor analyte values is greater than a predetermined number of sensor analyte values. Additionally or alternatively, the set duration may be a period of time selected from between 24 hours to 72 hours, specifically 36 hours to 48 hours.

In embodiments, the high analyte target value may be a first blood glucose measurement value, specifically an upper boundary of a blood glucose value. The low analyte target value may be a second blood glucose measurement value, specifically a lower boundary of a blood glucose value.

In embodiments, the predetermined time increment may be selected based on a number of the predetermined time intervals. In examples, the predetermined time increment may be selected from 1 to 60 predetermined time intervals, specifically from 2 to 30 predetermined time intervals, and more specifically from 5 to 15 predetermined time intervals, for instance, 6 predetermined time intervals.

In embodiments, the predetermined time interval may be selected between 1 to 10mins, specifically between 2 to 5mins. The predetermined time interval may also be referred to as a cycle. In examples, the predetermined time interval may be a cycle during which a sensor provides one sensor value.

In embodiments, after the set duration, a drug delivery algorithm may be applied based on at least the obtained sensor analyte values and/or the modified initial basal amounts.

One or more of the above embodiments may be combinable with in any one or more of other embodiments.

In embodiments, a wearable drug delivery device is provided which comprises a reservoir, a delivery mechanism or pump mechanism, a processor and a memory. The reservoir may be operable to contain a liquid drug. The delivery mechanism or pump mechanism may be fluidly coupled to the reservoir. The memory may store instructions that, when executed by the processor, the processor is operable to perform the computer-implemented method as described in any one of the embodiments herein above.

In embodiments, a drug delivery system is provided. The drug delivery system may comprise a wearable drug delivery device, and a hand-held device. The wearable drug delivery device may comprise a reservoir that is operable to contain a liquid drug, a delivery mechanism or pump mechanism fluidly coupled to the reservoir, and a first processor configured to drive the delivery mechanism or pump mechanism. The hand-held device may comprise a second processor and a memory storing instructions that, when executed by the first and/or the second processor, cause the first and/or the second processor to perform the computer-implemented method as described in any one of the embodiments herein above. In embodiments, the drug delivery system may further comprise an analyte sensor configured to obtain sensor analyte values.

In a second aspect, a computer-implemented method for automatic adjustment of a setpoint for a target sensor analyte value is provided. The computer-implemented method comprises obtaining sensor analyte value related data over a period of time. The obtained sensor analyte value related data is compared to reference sensor analyte value related data. Based on the comparison, it is evaluated if an adjustment of the setpoint is needed. Based on the result of the evaluation indicating a need for adjustment, a modification operation of the previous setpoint for the target sensor analyte value is performed. In embodiments, the setpoint for a target sensor analyte value (also referred to as "setpoint") may typically be one specific value, for instance a target blood glucose level. However, it should be understood that in some embodiments, the setpoint may include two or more target values, for instance two boundary values.

In embodiments, the sensor analyte value related data may comprise data of sensor analyte values and/or data of drug deliveries.

In embodiments, comparing may be performed at predetermined time intervals within the period of time.

In embodiments, the sensor analyte value related data may comprise data of sensor analyte values and comparing may comprise comparing a respective sensor analyte value to a high target analyte value and to a low target analyte value. Maintaining a high target count of a number of the respective sensor analyte values being greater than the high target analyte value. Maintaining a low target count of a number of the respective sensor analyte values being less than the low target analyte value.

In embodiments, the evaluation may comprise evaluating the high target count against a high target count threshold and/or evaluating the low target count against a low target count threshold.

In embodiments, the high target count threshold is a high target percentage of time threshold. Additionally, evaluating the high target count against the high target count threshold may further comprise determining relative to the period of time, a percentage of time during which the sensor analyte values are greater than the high target value exceeds the high target percentage. In embodiments, the modification operation may further comprise in response to the determined percentage of time exceeding the high target percentage, decreasing the setpoint for the target sensor analyte value by a preset setpoint value. In embodiments, decreasing the setpoint for the target sensor analyte value by the preset setpoint value may be based on a number of percentage increments that the determined percentage of time is greater than the high target percentage. In embodiments, the modification operation may comprise decreasing the setpoint of the target sensor analyte value by a preset setpoint value for each percentage increment over a percentage of time that the high target count is greater than the high target count threshold.

In embodiments, the low target count threshold may be a low target percentage of time threshold, and wherein evaluating the low target count against the low target count threshold may further comprise determining relative to the period of time, a percentage of time during which the sensor analyte values are less than the low target value exceeds the low target percentage. In embodiments, the modification operation may further comprise in response to the determined percentage of time exceeding the low target percentage, increasing the setpoint for the target sensor analyte value by a preset setpoint value. In embodiments, increasing the setpoint for the target sensor analyte value by the preset setpoint value may be based on a number of percentage increments that the determined percentage of time is greater than the low target percentage.

In embodiments, the sensor analyte value related data may comprise data of drug deliveries, and wherein comparing may comprise comparing past drug deliveries and expected drug deliveries over a partial period of time. An adjustment may be needed if based on the comparison a deviation between the past drug deliveries and expected drug deliveries is evaluated. In embodiments, drug deliveries may comprise basal deliveries and/or bolus deliveries.

Additionally, the modification operation may further comprise in response to the deviation indicating that the past drug deliveries being less than the expected drug deliveries, increasing the setpoint for the target sensor analyte value by a preset setpoint value. Alternatively or additionally, the modification operation may further comprise in response to the deviation indicating that the past drug deliveries being greater than the expected drug deliveries, increasing the setpoint for the target sensor analyte value by a preset setpoint value. In embodiments, adjusting the setpoint for the target sensor analyte value by the preset setpoint value may be based on a number of percentage increments that the determined deviation deviates from the expected drug deliveries.

In embodiments, the period of time may be selected between 12 hours to 72 hours, specifically between 24 hours to 48 hours. Additionally or alternatively, the partial period of time may be selected between 1 hours to 6 hours, specifically between 2 hours to 5 hours.

In embodiments, after the modification operation, the adjusted setpoint may be evaluated against an upper setpoint boundary and/or a lower setpoint boundary. The adjusted setpoint may only then be set as new setpoint if it is within at least one of the upper setpoint boundary and the setpoint lower boundary.

One or more of the above embodiments may be combinable with in any one or more of other embodiments.

In embodiments, a wearable drug delivery device is provided which may comprise a processor, and a memory storing instructions that, when executed by the processor, configure the processor to perform the computer-implemented method of the second aspect according to any one of the embodiments described herein above.

In embodiments, a drug delivery system is provided which may comprise a wearable drug delivery device and a hand-held device. The wearable drug delivery device may comprise a reservoir that is operable to contain a liquid drug, a delivery mechanism or pump mechanism fluidly coupled to the reservoir, and a first processor configured to drive the delivery mechanism or pump mechanism. The hand-held device may comprise a second processor and memory storing instructions that, when executed by the first and/or the second processor, cause the first and/or the second processor to perform the computer-implemented method of the second aspect according to any one of the embodiments described herein above. In embodiments, the drug delivery system may further comprise an analyte sensor configured to obtain sensor analyte values.

In a third aspect, a method is provided that includes determining a user is initiating a wearable drug delivery device for an initial use. A drug delivery algorithm is adjusted, in response to the initial use, by setting a high analyte target value and a low analyte target value, to begin delivery of a basal amount of a liquid drug. The basal amount may be modified by a set amount of liquid drug at predetermined time increments within a set duration. After the set duration, the drug delivery algorithm may be readjusted according to respective sensor analyte values of a number of sensor analyte values stored during the set duration.

In a fourth aspect, a wearable drug delivery device is provided that includes a reservoir, a pump mechanism, a processor, and a memory. The reservoir is operable to contain a liquid drug. The pump mechanism is fluidly coupled to the reservoir. The memory stores instructions that, when executed by the processor, cause the processor to be operable to determine that a user is initiating the wearable drug delivery device for an initial use. The processor, in response to the initial use, may set a high analyte target value and a low analyte target value, and adjust a drug delivery algorithm to begin delivery of basal amount of a liquid drug. The basal amount may be modified by a set amount of liquid drug at predetermined time increments within a set duration. After the set duration, the drug delivery algorithm may be readjusted according to respective sensor analyte values of a number of sensor analyte values stored during the set duration.

In a fifth aspect, another method is provided that includes steps of receiving sensor analyte values over a period of time, at time of receipt of a respective sensor analyte value and comparing the respective sensor analyte value to a high target analyte value and to a low target analyte value. A high target count of a number of the respective sensor analyte values that are greater than the high target analyte value may be maintained and a low target count of a number of the respective sensor analyte values that are less than the low target analyte value may be maintained. The high target count may be evaluated against a high target count threshold and the low target count may be evaluated against a low target count threshold. Based on a result of either the evaluation of the high target count or the evaluation of the low target count indicating a need for a setpoint adjustment, a modification operation of a sensor analyte value setpoint may be performed.

In a sixth aspect, a wearable drug delivery device is provided that includes a processor, and a memory. The memory may store instructions that, when executed by the processor, configure the processor to receive sensor analyte values over a period of time. At time of receipt of a respective sensor analyte value, the respective sensor analyte value is compared by the processor to a high target analyte value and to a low target analyte value. The processor may maintain a high target count of a number of the respective sensor analyte values are greater than the high target analyte value, and a low target count of a number of the respective sensor analyte values are less than the low target analyte value. The high target count may be evaluated against a high target count threshold, and the low target count may be evaluated against a low target count threshold. Based on a result of either the evaluation of the high target count or the evaluation of the low target count indicate need for a setpoint adjustment, the processor may perform a modification operation of a sensor analyte value setpoint.

This summary is provided to introduce a selection of concepts in a simplified form that are further described below in the Detailed Description. This summary is not intended to identify key features or essential features of the claimed subject matter, nor is it intended as an aid in determining the scope of the claimed subject matter.

BRIEF DESCRIPTION OF THE DRAWINGS

In the drawings, like reference characters generally refer to the same parts throughout the different views. In the following description, various examples are described with reference to the following drawings, in which:

FIG. 1 illustrates a functional block diagram of an exemplary system suitable for implementing the systems, processes and techniques disclosed herein.

FIG. 2 illustrates an example process in accordance with the disclosed subject matter.

FIG. 3 illustrates another example process in accordance with the disclosed subject matter.

DETAILED DESCRIPTION

One of the benefits of the disclosed systems and techniques is the ability of a wearable automatic insulin device system to automatically adjust to the user's true insulin needs without the user having to remember to enter a target sensor analyte value setpoint and also calculate any adjustments to the target sensor analyte value set point.

Another benefit provided by the disclosed systems and processes are techniques that allow users to initialize their wearable drug delivery device with a single basal value that is determined by an automatic insulin device or medication delivery algorithm that controls, or provides control signals to, the wearable drug delivery device.

In the described examples, a type of medication delivery algorithm (MDA) may use an application that includes or implements an "artificial pancreas" algorithm-based system, or more generally, an artificial pancreas (AP) application, also referred to as an "AP application." Instances of an AP application may be configured to provide automatic delivery of insulin based on an analyte sensor input, such as signals received from an analyte sensor, such as a continuous blood glucose monitor, or the like. The signals from the analyte sensor may include blood glucose measurement values, time stamps, a value of a previous measurement, or the like.

Systems, devices, computer-readable media, processes and techniques in accordance with the present disclosure are now described more fully hereinafter with reference to the accompanying drawings, where one or more examples are shown. The systems, devices, and methods may be embodied in many different forms and are not to be construed as being limited to the embodiments set forth herein. Instead, these examples are provided so the disclosure is thorough and complete, and may fully convey the scope of methods and devices to those skilled in the art. Each of the systems, devices, and methods disclosed herein provides one or more advantages over conventional systems, components, and methods.

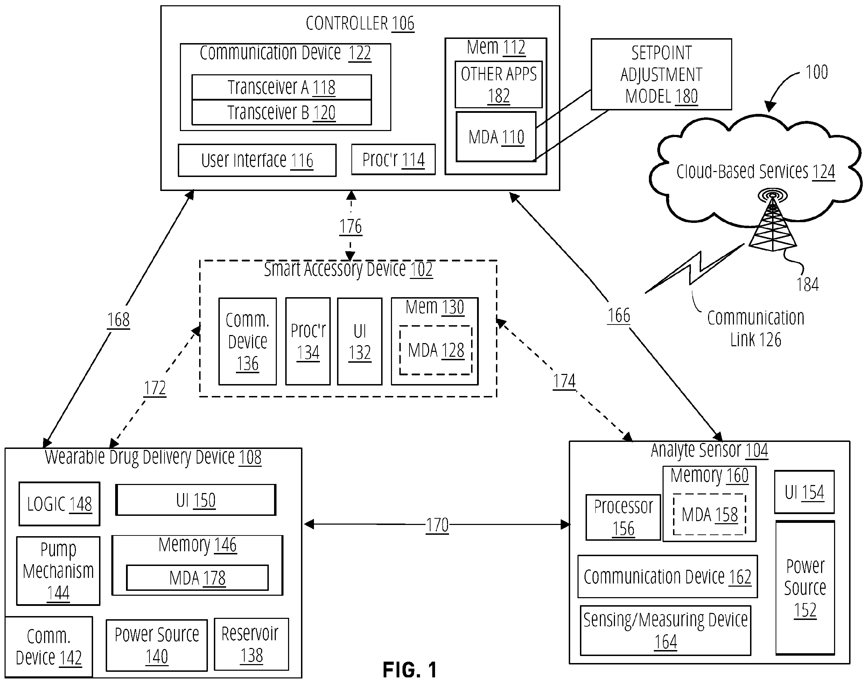

FIG. 1 illustrates a functional block diagram of a system example suitable for implementing the example processes and techniques described herein. The drug delivery system 100 may implement (and/or provide functionality for) a medication delivery algorithm, such as an artificial pancreas (AP) application, to govern or control the automated delivery of a drug or medication, such as insulin, to a user (e.g., to maintain euglycemia - a normal level of glucose in the blood). The wearable drug delivery system 100 may be an automated drug delivery system that may include a drug delivery device 102 (which may be wearable), an analyte sensor 104 (which may also be wearable, such as a continuous glucose monitor), and a controller 106. The smart accessory device 102 may be optional. Cloud-based services 124 may also provide functionality or access to data to the respective components, 102, 104 and 106 of the wearable drug delivery system 100.

The controller 106 may be remote from the wearable drug delivery device 108. It is envisioned that the controller is a smartphone, tablet computer, a dedicated programmable diabetes management device, or the like. The controller 106 may include a user interface 116, a communication device 122, a memory 112 (also referred to as the "controller memory"), and a processor 114. The user interface 116 is coupled to the processor 114 and is operable to receive inputs related to a physiological condition of a wearer, drug delivery requests (e.g., a bolus request), device status update requests (e.g., amount of liquid drug remaining, or the like, and provide the input to the processor 114. The controller 106 may include a user interface 116, which may be a keypad, a touchscreen display, levers, light-emitting diodes, buttons on the controller 106, a microphone, a camera, a speaker, a display, or the like, that is configured to allow a user to enter information and, in some examples, allow the controller 106 to output information for presentation to the user (e.g., alarm signals, exercise recommendations (e.g., exercise times and/or exercise intensity, and the like) based on the received input. The user interface 116 may provide inputs, such as a voice input, a gesture (e.g., hand or facial) input to a camera, swipes to a touchscreen, or the like, that processor 114 interprets by executing programming code stored in the controller memory 112.

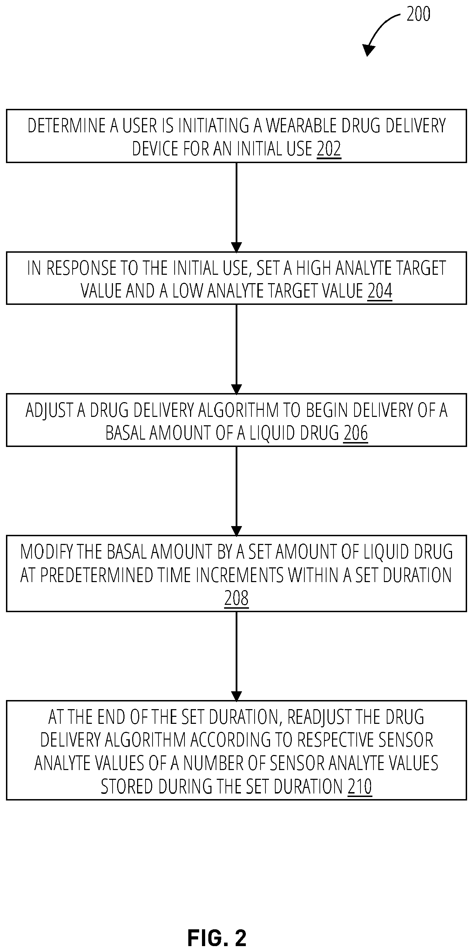

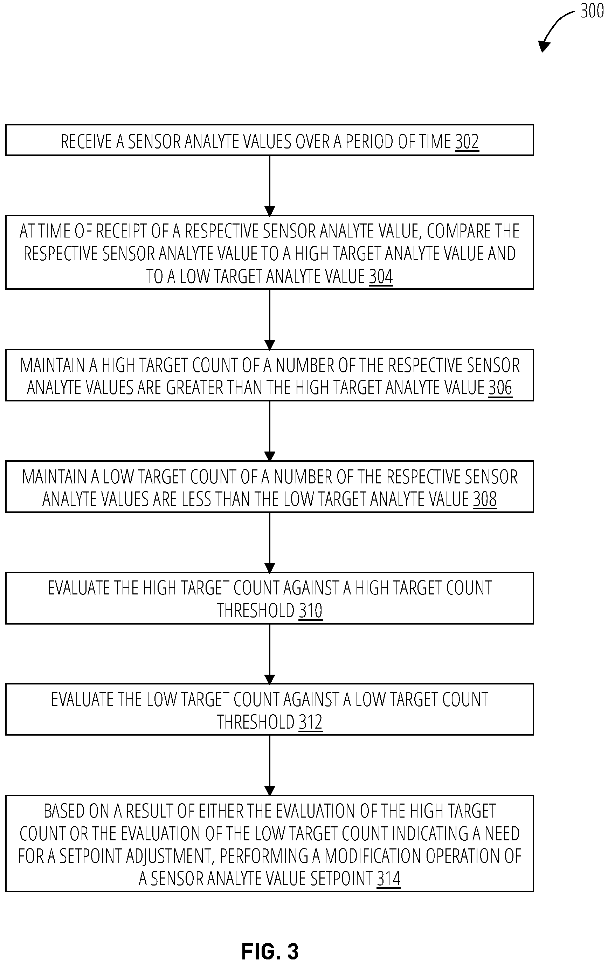

The controller 106 may contain analog and/or digital circuitry that may be implemented as a processor 114 for executing processes based on programming code stored in the controller memory 112, such as the medication delivery algorithm or application (MDA) 110 and/or the setpoint adjustment model 180 and related programming code. In addition, the memory 112 may store programming code, such as other apps 182 and MDA 110 as well as the setpoint adjustment model 180. For example, the processor 114 when executing the programming code may cause the controller to manage a user's blood glucose levels and control the delivery of a liquid drug, a medication, or a therapeutic agent to the user based on outputs from the MDA 110 and/or the setpoint adjustment model 180. The controller 106 may be used to program, adjust settings as discussed in the following examples, and/or control operation of the wearable drug delivery device 108 and/or the analyte sensor 104 as well as the optional smart accessory device 102. The setpoint adjustment model 180 may be programming code that when executed may cause the processor 114 to be operable to perform the functions and examples described with reference to FIG. 2 and FIG. 3.

The communication device 122 of the controller 106 may include one or more transceivers, such as transceiver A 118 and transceiver B 120, that may operate according to one or more radio-frequency protocols. In an example, the transceivers 118 and 120 may be a cellular transceiver and a Bluetooth® transceiver, respectively. For example, the transceiver A 118 or transceiver B 120 may be configured to receive and transmit signals containing information usable in the setpoint adjustment model 180, such as physiological conditions of a wearer, other physiological data, drug delivery amounts, and the like.

The wearable drug wearable drug delivery device 108 may include logic circuitry 148, a reservoir 138, a communication device 142, a power source 140, a memory 146, a user interface (UI) 150, and/or a pump mechanism 144. The logic circuitry 148 may be operable to control the drug delivery device. The reservoir 138 may be configured to contain a liquid drug. The communication device 142 may be coupled to the logic circuitry 148. The pump mechanism 144 may be responsive to the logic circuitry 148 and fluidically coupled to the reservoir 138.

In the example, the communication device 142, which may be a receiver, a transmitter, or a transceiver that operates according to one or more radio-frequency protocols, such as Bluetooth, Wi-Fi, a near-field communication standard, a cellular standard, or the like. The communication device 164 may enable the logic circuitry 148 to communicate with the controller 106 and the analyte sensor 104.

The wearable drug delivery device 108 may be attached to the body of a user, such as a patient or diabetic, at an attachment location and may deliver a liquid drug to a user at or around the attachment location. For example, a bottom surface of the wearable drug delivery device 108 may include an adhesive (not shown) to facilitate attachment to the skin of a user.

The reservoir 138 may store the liquid drug(s), medications or therapeutic agents suitable for automated delivery, such as diabetes treatment drugs (e.g., insulin, glucagon, glucagon-like peptides (e.g., GLP-1), insulin, pramlintide, co-formulations of two or more of GLP-1, pramlintide, and insulin or the like), pain relief drugs (e.g., morphine, a non-opioid or the like), hormones, blood pressure medicines, chemotherapy drugs, fertility drugs, or the like. The wearable drug delivery device 108 may include a needle and/or a subcutaneous cannula (not shown) coupled to the reservoir 138. When a needle deployment component of the drive mechanism 144 is activated, the needle and subcutaneous cannula may be operable to extend into the body of the user (e.g., subcutaneously, intraperitoneally, or intravenously) for delivering the liquid drug to the user. The pump mechanism 144 under control of the logic circuitry 148 transfers the liquid drug from the reservoir 138 through the needle or cannula and into the wearer based on control signals applied by the control logic or via the controller 106 or optional smart accessory device 102.

The power source 140, such as a battery, a piezoelectric device, other forms of energy harvesting devices, or the like, is configured to supply electrical power to the pump mechanism 144, the logic circuitry 148, the memory 146, and the communication device 142 of the wearable drug delivery device 108.

In some examples, the wearable drug delivery device 108 may include a user interface 150, which may be a keypad, a touchscreen display, levers, light-emitting diodes, buttons on a housing of the wearable drug delivery device 108, a microphone, a camera, a speaker, a display, a touchscreen display, or the like, that is configured to allow a user to enter information and allow the wearable drug delivery device 108 to output information for presentation to the user (e.g., alarm signals or the like). The user interface 150 may provide inputs, such as a voice input, a gesture (e.g., hand or facial) input to an optical sensor, swipes to a touchscreen, or the like, to processor 114 which the programming code interprets.

The optional smart accessory device 102 may be a smart watch, another wearable smart device, including eyeglasses, a global positioning system-enabled wearable device, a wearable fitness device, smart clothing, or the like, and may be operable to communicate with the other components of wearable drug delivery system 100 via wireless communication links communication link 172, 174, or communication link 176.

For example, the smart accessory device 102 may include a communication device 136, a processor 134, a user interface 132 and a memory 130. The user interface 132 may be a graphical user interface presented on a touchscreen display of the smart accessory device 102. The memory 130 may store programming code to operate different functions of the smart accessory device 102 as well as an instance of an MDA, MDA 128. The processor 134 that may execute programming code, such as MDA 128 for controlling the wearable drug delivery device 108 to implement the processes and techniques of FIGs. 2 and 3 described herein.

The analyte sensor 104 may include a processor 156, a memory 160, a sensing/measuring device 164, a user interface 154, a power source 152, and a communication device 162. The analyte sensor 104 may, for example, be a blood glucose monitor removably attachable via adhesive, for example, to a body of the wearer. In such an example, the analyte sensor 104 is operable to measure a blood glucose measurement value of the user/wearer and communicate with the controller 106 and the wearable drug delivery device 108 via the communication device 162 under the control of the processor 156. The memory 160 may be configured to store information and programming code, such as an instance of the MDA 158.

The analyte sensor 104 may be configured to detect multiple different analytes, such as lactate, ketones, uric acid, sodium, potassium, alcohol levels, blood glucose, proteins, hormones, or the like, and output results of the detections, such as measurement values or the like, for receipt by one or more of the smart accessory device 102, the controller 106, or the wearable drug delivery device 108. The analyte sensor 104 may, in an example, be configured to measure the blood glucose value at a predetermined time interval, such as every 5 minutes, or the like. The communication device 162 of analyte sensor 104 may have circuitry that operates as a transceiver for communicating the measured blood glucose values to the controller 106 over a wireless link communication link 166 or with the wearable drug delivery device 108 over the wireless communication link 170. While called an analyte sensor 104, the sensing/measuring device 164 of the analyte sensor 104 may include one or more additional sensing elements, such as a heart rate monitor, a pressure sensor, or the like.

The processor 156 of the analyte sensor 104 may be operable to perform many functions. For example, the programming code stored in the memory 160 may enable the processor 156 to manage the collection and analysis of data detected by the sensing and measuring sensing/measuring device 164, such as blood glucose measurement values, providing trend information and the like. The processor 156 may include discrete, specialized logic and/or components, an application-specific integrated circuit, a microcontroller or processor that executes software instructions, firmware, programming instructions stored in memory (such as memory 160), or any combination thereof.

The power source 152 may be a battery, a piezoelectric device, an energy harvesting device, or the like, for supplying electrical power to the components of analyte sensor 104.

Services provided by cloud-based services 124 may include servers and memory that stores anonymized data, such as blood glucose measurement values, historical insulin-on-board (IOB) or total daily insulin (TDI), maximum and minimum boundary values, therapeutic substance concentration values, substance sensitivity values, and other forms of data. In addition, the cloud-based services 124 may process the anonymized data from multiple users to provide generalized information related to TDI, insulin sensitivity, IOB, and the like. The cloud-based services 124 may be accessed by the controller 106, smart accessory device 102, analyte sensor 104 or wearable drug delivery device 108 via communication link 126 and data network device 184, which may be a Wi-Fi device, a cellular communication tower, a local area network, a campus wide network or the like.

The wireless communication links 126, communication link 166, 168, 170, 172, 174, and 176 may be any type of wireless link operating using known wireless communication standards or proprietary standards. As an example, the wireless communication links 166, 168, 170, 172, 174, and 176 enable communications based on Bluetooth®, Zigbee®, Wi-Fi, a near-field communication standard, a cellular standard, or any other wireless protocol via the respective communication devices 122, 136, 142, and 162.

Software related implementations of the techniques described herein, such as the processes examples described with reference to FIGs. 2 and 3 may include, but are not limited to, firmware, application specific software, or any other type of computer readable instructions that may be executed by one or more processors. The computer readable instructions may be provided via non-transitory computer-readable media. Hardware related implementations of the techniques described herein may include, but are not limited to, integrated circuits (ICs), application specific ICs (ASICs), field programmable arrays (FPGAs), and/or programmable logic devices (PLDs). In some examples, the techniques described herein, and/or any system or constituent component described herein may be implemented with a processor executing computer readable instructions stored on one or more memory components.

Various examples provide a method, a system, a device and a computer-readable medium for responding to inputs provided by sensors, such as an analyte sensor, and users of an automatic drug delivery system. The term "analyte sensor" may refer to a wearable device that includes sensing and measurement devices configured to detect different analytes in a user's body and particularly in the user's blood, as well as other analytes as described herein. The various devices and sensors that may be used to implement some specific examples may also be used to implement different therapeutic regimens using different drugs than those described in the specific examples.

The disclosed examples provide techniques that may be used with any additional algorithms or computer applications that manage blood glucose levels and insulin therapy. In addition, algorithms, such as the setpoint adjustment model 180 of FIG. 1, may be used to change settings related to the delivery of drugs such as diabetes treatment drugs (e.g., insulin, glucagon and/or glucagon-like peptides) as well as other categories of drugs, such as chemotherapy drugs, pain relief drugs, blood pressure medication, or the like.

FIG. 2 illustrates an example process of operation of a drug delivery algorithm at the initiation of use of wearable drug delivery system of a user.

The following discussion of process 200 describes a specific example for automatically adjusting an AID user's glucose control targets, and thereby providing the advantage of eliminating or reducing the need for diabetic users to define their own glucose control targets. While the specific example describes a diabetes related example, the following process is also applicable to drug delivery algorithms that control the delivery of drugs other than diabetes-related drugs based on the received sensor analyte values.

The process 200 may be implemented by a system such as the wearable drug delivery system 100 shown in FIG. 1, is described. The typical and edge cases of the operating margins for the various parameters used in AID systems are usually clinically validated. For example, a target blood glucose setpoint value between 70 mg/dL and 180 mg/dL is considered to be a typical glucose target setting for most users as validated through various clinical tests.

In block 202, when programming code that implements the process 200 is executed by a processor, the processor is operable to determine that a user is initiating a wearable drug delivery device for an initial use. The processor when determining that the user is initiating the drug delivery device for the initial use, may be operable to receive an input from a user input device indicating that the user is a new user. For example, the processor may receive a signal from a controller, such as controller 106 of FIG. 1, indicating that the user is a new user. Or the processor may receive a signal from a wearable drug delivery device in some instances, the processor may be operable via a communication interface, for example, to establish a wireless communication connection (e.g., a Bluetooth pairing, communication link via WiFi or a cellular data connection, or the like) with a controller device, and receive a signal from the controller device indicating the user is a new user. The signal from the controller may be automatically generated by a computer application implementing an AID algorithm or a drug delivery algorithm and communicatively coupled to the wearable drug delivery device or based on a user input solicited by the computer application.

In block 204, in response to the determination that the impending use of the wearable drug delivery device is an initial use of the wearable drug delivery device, the process 200 sets a high analyte target value and a low analyte target value. For example, the high analyte target value may be a first blood glucose measurement value, such as 158 mg/dL, 175 mg/dL or 180 mg/dL or the like, and the low analyte target value is a second blood glucose measurement value, such as 70 mg/dL, 75 mg/dL, 100 mg/dL or the like.

Although not shown in FIG. 2, the processor, for example, in response to the determination that the impending use of the wearable drug delivery device in the initial use, may be further operable, to select a basal amount from a default basal amount stored in a memory coupled to the processor. For example, the basal amount may also be set in response to a control signal from a controller, or the like. However, the setting of basal amount does not require that the processor have any demographic information regarding the user. In addition, the user's blood glucose measurement history is also considered insufficient and therefore does not influence the selection of the basal amount.

The basal amount is a number of units of the liquid drug delivered per a time increment. For example, the initial basal amount may be a default basal amount that has a value that has been selected based on clinical trials. Basal amounts are typically expressed in Units per hour (U/hr), for example, 0.2 U/hr. However, the number of units may range from 1.0 units to 3.00 units and the time increment may range from 0.5 hours to 3.00 hours. In an example, the default basal amount may be 1.00 unit and the time increment may be 3.00 hours, which converts to an hourly basal amount of 0.333 U/hr. Of course, the number of units may range over different values, such as 0.5 -4.0, or another time increment may also range over different values, such as 0.2-6.0 hours.

The process 200, in block 206, causes the processor to adjust a drug delivery algorithm to begin delivery of the basal amount of a liquid drug. For example, the processor may adjust the drug delivery algorithm, such as an MDA or AP application, to begin delivering the liquid drug according to the basal amount, which may be presented as a rate, such as the 0.2 U/hr mentioned above. For example, a pump mechanism of the wearable drug delivery device may be operable to deliver a preset amount of the liquid drug during a stroke of the pump mechanism. The preset amount may be the maximum capacity of the pump mechanism, such as 0.05 Units, per stroke. The pump mechanism is operable to provide several strokes within a time period to meet the set basal amount. For example, the pump mechanism may be operable to provide four strokes that each deliver 0.05 Units within one hour to satisfy the setting of 0.200 U per hour.

In block 208, the process 200 modifies the initial basal amount by a set amount of liquid drug at predetermined time increments within a set duration. In some examples, the set duration may be a period of time over which a number of sensor analyte values are stored. When, for example, the number of sensor analyte values is greater than a predetermined number of sensor analyte values, such as 700 or 864 or the like, the set duration ends. In another example, the set duration is a period of time selected from: 24 hours, 48 hours or 72 hours.

The set amount of the liquid drug may be a tunable amount or a predetermined amount of the liquid drug. For example, the set amount of the liquid drug indicated by the modification is equal to at least 0.05 units of the liquid drug. In the example, the 0.05 units of liquid drug may be a maximum amount of liquid drug that may be delivered by each pump stroke or cycle of the wearable drug delivery device. Alternatively, the set amount may be a variable amount based one or more factors, such as rate of change of the sensor analyte value, magnitude of the change of the sensor analyte value, or the like. For example, the set amount may be lower than 0.05, such as 0.010, or higher than 0.05, such as 0.15, or the like. In examples, the set amount may range between 0.005 and 0.2 units of the liquid drug, specifically between 0.01 and 0.15 units of the liquid drug. In specific embodiments, the set amount may be at least 0.005 units of the liquid drug or at least 0.05 units of the liquid drug.

The basal amount $B_{init}(i)$ may be set according to a specific equation. The basal amount $B_{init}(i)$ may also be referred to as an hourly basal insulin need for a particular user, and the terms "basal amount" and "hourly basal insulin need" may be used interchangeably. As shown in the following equation, a number of different parameters may be tuned to adjust the basal amount based on inputs from the analyte sensor. In Equation 1:

$$B_{init}(i) = 0.15 + \min\left(0.05 \cdot \max\left(1 + \text{floor}\left(\frac{\text{N}_{G \geq Hi\,Target}}{\text{No. Cycles}}\right) - \text{ceil}\left(\frac{\text{N}_{G \leq Lo\,Target}}{\text{No. Cycles}}\right), 1\right), 1\right) \quad \text{(Eq. 1)}$$

where $N_{G \geq Hi\,Target}$ is the number of cycles that the user's blood glucose value is greater than the Hi Target value (which is a high analyte target setting made at block 204), $N_{G \leq Lo\,Target}$ is the number of cycles that the user's blood glucose value is less than the Lo Target value (which is a low analyte target setting that is also made at block 204), and No. Cycles is the number of cycles in which the drug deliver algorithm (e.g., MDA) receives a sensor analyte value from an analyte sensor. In an example, a cycle may last approximately 5 minutes, although other time periods, such as less than 1 minute, 1 minute, 10 minutes or the like may also be used. An analyte sensor typically provides a sensor analyte value at set intervals, e.g., every 5 minutes. However, some analyte sensors are capable of providing sensor analyte values more frequently, such as every minute, so Equation 1 may be modified by replacing the No. Cycles variable with a set number of sensor analyte values or a specific time.

In Equation 1, the maximum (Max) function outputs the maximum value between a value equal to (0.05 times the value of 1 plus an output of a floor function (for example, a function that rounds down an input to a nearest integer) minus an output of a ceil[ing] function (for example, a function that rounds up an input to the nearest integer) and the integer 1. A minimum (Min) function selects the minimum value between the output of the Max function multiplied by 0.05 and the integer 1. The output of the Min function may be summed with the value 0.15 to provide the basal amount $B_{init}(i)$. In some examples, the typical value for the basal amount $Binit(i)$ may be 0.2 U/hr.

The set amount of the liquid drug indicated by the modification may either be a positive set amount or a negative set amount. The determination of whether the set amount is positive is based on the number of sensor analyte values that are greater than the high target setting during a predetermined time increment. The predetermined time increment may be based on a number of cycles of the analyte sensor or after a set number of times the analyte sensor has provided a sensor analyte value to the processor.

For example, the set amount of the liquid drug indicated by the modification is a positive set amount when the sensor analyte value is greater than the high analyte target value more often than being below the high analyte target value over the course of the predetermined time increment. Alternatively, the determination of whether the set amount is negative is based on the number of sensor analyte values that are less than the low target setting during a predetermined time increment. For example, the set amount of the liquid drug indicated by the modification is a negative set amount when the sensor analyte value is less than the low analyte target value more often than being above the low analyte target value over the course of the predetermined time increment.

The processor at block 208 may determine the modification of the basal amount and any subsequent amounts at predetermined time increments, such as every 3 hours, 5 hours, 6 hours, 8 hours, 12 hours, or the like. The modification may be determined using Equation 1, where $B_{init}(i)$ becomes a modified basal amount.

In a specific example using hypothetical but realistic tunable parameters inserted in Equation 2 shows:

$$B_{init}(i) = 0.15 + \min\left(0.05 \cdot \max\left(1 + \text{floor}\left(\frac{N_{G \geq 180}}{6}\right) - \text{ceil}\left(\frac{N_{G \leq 70}}{6}\right), 1\right), 1\right),$$

where (comparing to Eq. 1 above) Hi Target is set to 180 as in 180 mg/dL, Lo Target is set to 70 as in 70 mg/dL, the No. Cycles is set to 6, which equates to 30 minutes (i.e., 5 minutes per cycle), $N_{G>=180}$ represents the number of cycles during which the user's glucose is above 180 mg/dL, and $N_{G<=70}$ represents the number of cycles during which the user's glucose is below 70 mg/dL.

In an operational example, the basal input begins at a reasonable minimal value - 0.2U/h - and is increased by 0.05U for every 30 minutes the user's glucose is above 180 mg/dL or decreased by 0.05U for every 30 minutes the user's glucose is below 70 mg/dL. This basal input may range between 0.0 U/h and 1 U/h, during a set duration of time. The processor may maintain in a memory a count of the number of times the sensor analyte value was above the high target setting and the number of times the sensor analyte value was below the low target setting for the selected number of cycles (i.e., No. Cycles). In the example, the floor and ceiling functions provide rounding of values to the lowest, or highest, whole number, respectively. For instance, if N is between 1.00-1.99, the floor function would return a 1, while the ceiling function would return a 2. In the example, the MDAs may be operable to arrive at the maximum basal of 1U/h, if the user is above 180 mg/dL for longer than 8.5 hours. In addition, the MDA may be tuned to be more aggressive by increasing the set amount, for example, from 0.05 U to 0.10 U. In addition, the basal input or basal amount may be increased from an initial amount of 0.2 U/hour to 0.4 or 0.6 U/hour. Furthermore, during the initialization phase, the MDAs may still suspend delivery of the liquid drug, when the user's sensor analyte value remains low.

The set duration may also be referred to as an "initialization phase." During the initialization phase, the MDA is initialized to control delivery of the liquid drug based on the number of sensor analyte values obtained during the set duration. As mentioned above, the set duration is based on the number of sensor analyte values stored beginning from when the wearable drug delivery device starts to deliver the liquid drug to the user.

If the wearable drug delivery device is operating properly and the number of sensor analyte values are stored properly, the AID/MDA algorithm is operable to consider the stored number of sensor analyte values as a sufficient user drug delivery history. A sufficient user drug delivery history enables the AID/MDA algorithm to use the sensor analyte values and the related information to determine a liquid drug basal delivery schedule with greater accuracy as well as when to recommend bolus dosages and an amount for a respective bolus dose. A user drug delivery history may be determined to be sufficient if it includes data from 48 hours out of 56 hours of use of a wearable drug delivery device. Of course, other numbers of hours, such as 24, 36 or 72, may be used instead of 48 hours.

Note that this implementation of the user's basal amount is designed to be active only during the initial period of use of the system (i.e., initialization phase) when insufficient insulin delivery history is available (i.e., the number of sensor analyte values does not meet a threshold, such as 700, 864, or the like or the set number of hours (e.g., 48 or 72 hours) of data history is not available), and it is expected that the system may be operable to revert to basal delivery as estimated by the actual insulin delivery history once a sufficient history (e.g., 72 hours) is available.

The AID/MDA algorithm, when executing during the initialization phase, may also modify an initial set point ($SP_{init}(i)$), which the system's control target, over time. For example, as shown in Equation 3 below, the lowering from 180 mg/dL to 110 mg/dL over 72 hours (i.e., the set duration), modified by the user's sensor analyte values, such as glucose concentrations, above 140 mg/dL or below 80 mg/dL may be calculated as follows (Equation 3):

$$SP_{init}(i) = 180 - 70 \cdot \min\left(1, \max\left(0, \frac{N_{active} + N_{G \geq 140} - 3 \cdot N_{G \leq 80}}{72 \cdot 12}\right)\right) \qquad (\text{Eq. 3})$$

where $N_{active}$ is a number of cycles that the wearable drug delivery device has operated and received a sensor analyte value, $N_{G \geq 140}$ is a number of cycles that the sensor analyte value has been greater than 140 mg/dL, and $N_{G \leq 80}$ is a number of cycles that the sensor analyte value has been below 80 mg/dL.

In this example, the values 80 and 140 are clinically determined high/low target settings for a user's high/low sensor analyte values. A blood glucose measurement value within the glycemic range of 80 mg/dL and 140 mg/dL may be considered to be within a safe glycemic range, but other values, such as 70 mg/dL and 180 mg/dL or the like, may be used. These high/low target settings may be tuned or modified by the user or AID/MDA algorithm. The values 72 and 12 in the denominator are also tunable. The value "72" represents the number of cycles that occur in a 6 hour period (12 cycles/hr times 6 = 72) and the number 12 is the number of hours corresponding to basal delivery. The value 3 in the numerator is a multiplier that enables the number of times the sensor analyte value is less than the low target setting to be weighed more heavily. For example, in the specific example of Equation 3, the number of cycles where the user's sensor analyte value, such as blood glucose concentration, is above 140 mg/dL doubles the rate of reduction of the target setting, and the number of cycles where the user's sensor analyte value is below 80 mg/dL slows the rate of increase of the target setting by 4 times.

The particular implementation of the setpoint shown in Equation 3 does not necessarily need to be limited to the initialization phase and can be utilized continuously as a dynamic setpoint outside the 72 hour window example for the initialization phase. In addition, the setpoint of Equation 3 may be used continuously to calculate a new setpoint or an update of the user's setpoint even when there is a lack of user's insulin delivery requests. Equation 3 may be used to set the setpoint ($SP$) in real time over the 72 hours (or whatever the selected time frame) of the initialization phase.

Returning to the example of FIG. 2, in block 210, process 200 after the set duration, readjusts the MDA according to respective sensor analyte values of a number of sensor analyte values stored during the set duration. For example, the number of sensor analyte values may be sufficient enough that the AID/MDA is operable to cause a processor to analyze the number of sensor analyte values, the amount of liquid drug delivered over the set duration, and/or other factors to determine variable amounts of the liquid drug that more effectively maintain the user within range (e.g., $\pm$ 10%) of the user's target blood glucose setpoint. Using the determined variable amounts, the liquid drug may be delivered to the user accordingly. Utilizing the techniques and process described with reference to FIG. 2, the AID/MDA more effectively maintains the user's target blood glucose measurement (i.e., blood glucose concentration) value substantially within a predetermined range of the analyte target blood glucose setpoint value. The "other factors" may include a time stamp of when the sensor analyte value was obtained, the time of delivery of the liquid drug, and the like.

In a further example, the processor may be operable to inform the user that the AID/MDA algorithm may behave conservatively during the initialization phase and provide prompts on a user's controller encouraging the user to deliver additional boluses as necessary. These additional boluses may initially be automatically calculated by the AID/MDA and quantified as follows to allow for increases in their delivery over time. For example, using Equation 4 below, the AID/MDA algorithm set during the initialization phase may be operable to set boluses without user input.

In the example, the automatic calculation of an additional bolus amount in Units may be as shown in Equation 4 below:

$$U_{auto,init}(i) = \left(0.005 \cdot \frac{N_{G \geq 180}}{6}\right) \cdot B_{init}(i) \cdot 48 + \frac{\frac{(G(i) - 180)}{1800} - IOB(i)}{B_{init}(i) \cdot 48},$$

where $\dfrac{\frac{(G(i) - 180)}{1800} - IOB(i)}{B_{init}(i) \cdot 48}$ is a correction factor in which $G(i)$ is the sensor analyte value for the cycle i and accounts for over or under delivery of insulin. The factor $\dfrac{1800}{B_{init}(i) \cdot 48}$ is based on an 1800 Rule combined with converting the current hourly basal value into TDI, by multiplying this parameter by 48. Note that the 1800 value may be tuned and may be some other value such as 1600, 2000, or the like. The user's current glucose concentration G(i) above the threshold for hyperglycemia, 180 mg/dL, is converted into an increase or a reduction in the insulin requirements depending on the user's glucose concentrations (e.g., blood glucose measurement values). The user's hourly basal insulin needs $B_{init}(i)$ is also converted to the daily insulin needs via multiplying by 48, and further multiplied by the factor

$0.005 \cdot \dfrac{N_{G \geq 180}}{6}$, where $N_{G \geq 180}$ is the number of 5-minute periods that the user's blood glucose measurement have been greater than or equal to 180 mg/dL since the start of the use of the device, divided into 30 minute cycles (represented by the value 6 as the number of 5-minute control cycles of the drug delivery device). The result of Equation 4 is the automatically determined, and an initial Units ($U_{auto,init}(i)$) is provided to the user if the user requests a bolus. This automatically determined, initial units $U_{auto,init}(i)$ may also be limited to the initialization phase, and alternate examples of the equation 4 can be utilized when sufficient insulin delivery history (also referred to as "user drug delivery history"), such as 72 hours, is available. For example, the value 72 may be substituted for the 48 and the like.

For instance, a user's initialized basal insulin need, $B_{init}(i)$ may be 0.6 U/h, and the user's current glucose concentration may be 240 mg/dL, with an IOB of 0.1U. The user may have experienced glucose above 180 mg/dL for 48 cycles, or 4 hours, since the start of the use of the device. The first term of equation 4 results in

$$0.005 \cdot \frac{48}{6} \cdot 0.6 \cdot 48 = 1.152 \text{U}$$

. The additional correction term results in $\dfrac{\frac{240-180}{1800}}{0.6 \cdot 48} = 0.96\text{U}$ . Finally, the IOB is 0.1U, for an overall equation of $1.152U + 0.96U - 0.1U = 2.012$U which will be delivered to the user if the user requests an insulin bolus.

In an example, the processor may also cause the presentation of a prompt indicating that the bolus amount may be manually modified with a further notification encouraging the number of manual overrides of the automatically determined, initial bolus amount $U_{auto,init}(i)$ be limited.

FIG. 3 illustrates another example process of operation of an AID/MDA at the initiation of use of wearable drug delivery system of a user. For example, programming code may be stored in a memory, such as memory 112, memory 130, or memory 146 as shown in FIG. 1, that may be executed by a processor to provide the process 300. The process 300 may be implemented by an MDA and/or a rapid adaptivity model, such as 180 of FIG. 1. Although shown as part of the MDA 110 of FIG. 1, the Setpoint adjustment model 180 may be implemented by the MDA 128 or MDA 178 or a combination of both. The inclusion of the setpoint adjustment model 180 as part of MDA 110 is shown for illustration and not intended to be a limitation on the implementation of the examples disclosed herein.

In the example process 300, when a wearable drug delivery device is initialized a default set point (i.e., target analyte value) for an analyte setting (e.g., a blood glucose setting) would be selected thereby allowing a user to skip having to input a desired set point and avoid the potential of entering an incorrect or inappropriate set point.

In block 302, the process 300 may receive sensor analyte values over a period of time. In the process 300, the period of time is one of: 12 hours, 24 hours, 36 hours, or 48 hours. In some examples, a processor may select the period of time based on different criteria. For example, a period time of a shorter duration, such as 12 hours or less, may be selected when a user's sensor analyte value remains substantially constant, such as within 5-10% of a target set point for most (e.g., where most is approximately 85-95% or the like) of the 12 hour period. Alternatively, a period time of a longer duration may be selected when a user's sensor analyte value is inconsistent, such as when the user's sensor analyte values stay with 5-10% of a target set point for only brief amounts of time, such as 1-10% or the like, of a longer period of time, such as a 12, 24, 36 or 48 hour period of time.

In block 304, when performing the process 300, a processor may be operable, at time of receipt of a respective sensor analyte value, to compare a respective sensor analyte value to a high target analyte value and/or to a low target analyte value. In some instances, the risks associated with hypoglycemia may outweigh the risks associated with hyperglycemia so the processor may evaluate each respective sensor analyte value against the low target analyte value more frequently than against the high target analyte value. For example, each respective sensor analyte value may be evaluated against the low target analyte value upon receipt, while some predetermined number of sensor analyte values, such as every 5 or 6, may be evaluated against the high target analyte value.

In block 306, the processor may maintain in a memory a high target count of a number of the respective sensor analyte values that are greater than the high target analyte value.

In block 308, the processor may maintain in the memory a low target count of a number of the respective sensor analyte values that are less than the low target analyte value.

In block 310, process 300 evaluates the high target count against a high target count threshold. A processor when evaluating the high target count against the high target count threshold, may be further operable to determine a percentage of time that the sensor analyte values are greater than the high target count threshold exceeds a high target percentage. The high target percentage (also referred to as "high target percentage of time threshold") is a percentage threshold (e.g. a time threshold expressed in percentage relative to the total period of time) above which high target counts may result in an adjustment of the setpoint. More specifically, the high target percentage is a lower boundary of percentage of time compared to the total period of time during which the obtained sensor analyte values are greater than the high target value. The determined percentage of time (regarding high target counts) is that percentage of time which exceeds the high target percentage.

In block 312, process 300 evaluates the low target count against a low target count threshold. The method may also include where evaluating the low target count against the low target count threshold, further includes determining a percentage of time that the sensor analyte values are less than the low target value exceeds a low target percentage.

The low target percentage (also referred to as "low target percentage of time threshold") is a percentage threshold (e.g. a time threshold expressed in percentage relative to the total period of time) above which low target counts may result in an adjustment of the setpoint. More specifically, the low target percentage is a lower boundary of percentage of time compared to the total period of time during which the obtained sensor analyte values are less than the low target value. The determined percentage of time is that percentage of time (regarding low target counts) which exceeds the low target percentage.

In block 314, the processor when executing process 300 may perform, based on a result of either the evaluation of the high target count or the evaluation of the low target count indicating need for a setpoint adjustment, a modification operation of a sensor analyte value setpoint. The modification operation may include an operation to adjust the setpoint of the target sensor analyte value. An adjustment of the setpoint of the target sensor analyte value may include increasing or decreasing the setpoint of the target sensor analyte value.

In an example, a processor, when performing the modification operation may be operable to, in response to the determined percentage of time that the user's sensor analyte value exceeded the high target percentage, decrease a setpoint for a target sensor analyte value by a preset setpoint value. The processor when decreasing of the setpoint for the target sensor analyte value by the preset setpoint value may perform the decrease based on a number of percentage increments that the determined percentage of time is greater than the high target percentage. Said differently, the modification operation of the sensor analyte value setpoint may include decreasing the setpoint by a preset setpoint value for each percentage increment over a percentage of time that the high target count is greater than the high target count threshold.

In another example, the processor may perform the modification operation in response to a determined percentage of time that the user's sensor analyte value remained below a present sensor analyte value setpoint being less than a low target percentage. For example, the processor may increase the sensor analyte value setpoint for a target sensor analyte value by a preset setpoint value. In some examples, increasing the setpoint for the target sensor analyte value by the preset setpoint value is based on a number of percentage increments that the determined percentage of time is less than the low target percentage.

In an operational example implementing the process 300, a user's sensor analyte value may be set to a target analyte value, which in this example is a target blood glucose measurement value (but also referred to as "blood glucose," "blood glucose value," "blood glucose setting," or "blood glucose set point"). For example, the target analyte value can be automatically targeted to a default value, $SP_d$ at initial system start. The processor may adjust the target periodically based on the outcomes of the user's % times in specific analyte value ranges (or, in the case of a diabetic patient within blood glucose measurement value ranges obtained from an analyte sensor).

The processor may use various techniques to determine the amount of modification or adjustment. For example, the target can be adjusted periodically based on the outcomes of the user's percentage of time in specific glucose ranges, as follows in Equation 5:

$$SP_{new}(i) =$$

$$\max\left(\min\left(SP_d - 10\left(\left(\frac{max\left(0, \frac{n_{g>180}}{288} - 0.15\right)}{0.05}\right) - \left(\frac{max\left(0, \frac{n_{g<70}}{288} - 0.015\right)}{0.005}\right)\right), SP_{ub}\right), SP_{lb}\right)$$

(Eq. 5), where $SP_d$ is the default set point (for example, 110 mg/dL), $n_{g>180}$ is the number of cycles that the sensor analyte value for blood glucose was greater than 180 mg/dL (which is an example value for the high target analyte value), $n_{g<70}$ is the number of cycles that the sensor analyte value for blood glucose was less than 70 mg/dL (which is an example value for the low target analyte value), the $SP_{ub}$ is an upper boundary for the set point (also referred to as "upper setpoint boundary $SP_{ub}$" or "setpoint upper boundary $SP_{ub}$")) that may be higher than (or lower than) the high target analyte value; the $SP_{lb}$ is a lower boundary for the set point (also referred to as "lower setpoint boundary $SP_{lb}$" or "setpoint lower boundary $SP_{lb}$") that may be lower than (or greater) the low target analyte value; the value 10, which is a maximum amount of adjustment (either increase/positive or decrease/negative) in Equation 5, may be a tunable variable (selectable based on user demographics or preferences), the value 288 is the number of cycles of the wearable drug delivery system within a 24 hour period of time. In Equation 5 example, each cycle is set to be 5 minutes, which also corresponds to how often the analyte sensor, such as 104 of FIG. 1, outputs a sensor analyte value (e.g., blood glucose measurement). Of course, a cycle may have a longer or shorter duration than 5 minutes. A cycle may also be referred to as a predetermined time interval at which a sensor value is obtained and/or compared. In examples, the predetermined time interval (i.e. cycle) may be between 1 min to 10 mins, specifically 1 min to 5mins, and more specifically 2, 3, 4 or 5 mins.

In the operational example, if the percentage of time the user is in the hyperglycemic range within the last 24 hours exceeds a predetermined percentage (i.e. high target percentage, e.g. hyperglycemic range percentage), the setpoint may be modified or adjusted. The predetermined percentage may generally also be referred to as "percentage threshold" and/or specifically regarding high target values "high target percentage threshold". In addition, the processor may determine that for every hyperglycemic range percentage increment, such as 5% of time user's blood glucose measurement value is above 180mg/dL, the setpoint is adjusted downwards. In a specific example, the user may have a hyperglycemic range percentage of 15%, in which case the setpoint may be modified or adjusted by a maximum amount of adjustment, which in this example is decreasing the setpoint by 10 mg/dL for each 5% above 15%, where the tunable value 10 is the maximum amount of adjustment value in the equation 5 above. Of course, the tunable value 10 may be set by a user or clinician and values different from 10 may be selected, e.g. at a value between 5 to 15. The tunable value may also be referred to as preset setpoint value. Similarly, the percentages may be selected from a range of percentages (i.e., percentage increment and/or percentage threshold) such 3-5% or 5-10% or 10-25% or the like. For instance, the percentage threshold may be selected from a range of percentages between 0.5%-5%, specifically 1%-4% and more specifically 2%-3%. In specific examples, the percentage threshold may be set to 1.25%, 1.5%, 1.75%, or 2.5%. In examples, the percentage increment may be selected from a range of percentages between 0.1%-1.5%, specifically 0.2% to 1.25% and more specifically 0.25% to 1%. In specific examples, the percentage increment may be set to 0.25%, 0.5%, 0.75%, or 0.8%.

Similarly, the setpoint may be increased based on the result of Equation 5 when the percentage of time the user is in the hypoglycemic range within the last 24 hours exceeds a predetermined percentage (i.e. low target percentage, e.g. hypoglycemic range percentage). The predetermined percentage may generally also be referred to as "percentage threshold" and/or specifically regarding low target values "low target percentage threshold". Since there are more significant health effects from the user being hypoglycemic, the hypoglycemic range percentage is typically lower than the hyperglycemic range percentage. For example, the setpoint may be increased by 10 mg/dL for each 0.5% time in the hypoglycemia above 1.5%. The hypoglycemic range percentage (1.5%) and hypoglycemic range percentage increment (0.5%) may be, for example, 10 times more sensitive for hypoglycemia versus hyperglycemia because hypoglycemia is a greater risk. Of course, the 10 time more sensitive setting may be tunable as well and may be set at a value between 5 to 15, e.g. 5, 15, 20 or the like. The tunable value may also be referred to as preset setpoint value. This evaluation based on Equation 5 may be executed periodically, such as every 24 hours or the like. Similarly, the percentages (i.e., percentage increment and/or percentage threshold) may be selected from a range of percentages such 1.3-1.5% or 2-4% or 0.25-0.75%, 0.8% or the like. For instance, the percentage threshold may be selected from a range of percentages between 0.5%-5%, specifically 1%-4% and more specifically 2%-3%. In specific examples, the percentage threshold may be set to 1.25%, 1.5%, 1.75%, or 2.5%. In examples, the percentage increment may be selected from a range of percentages between 0.1%-1.5%, specifically 0.2% to 1.25% and more specifically 0.25% to 1%. In specific examples, the percentage increment may be set to 0.25%, 0.5%, 0.75%, or 0.8%.

Alternatively, the user's glucose target set point can also be automatically adjusted in real time based on the user's total insulin delivery over time. For example, the processor may be tuned to look at a period of time, such as the last 5 hours in which the user was using the wearable drug delivery device. In comparison to the period of time used with respect to equation 5, the period of time with respect to equation 6 may also be referred to as "partial period of time". The processor may evaluate the deviation between the amount of therapeutic to be should get based on TDI (also referred to as "expected drug deliveries") versus what the actual insulin delivery is (also referred to as "past drug deliveries"). The algorithm when implementing the technique of Equation 5 is looking at the last 5 hours and determining a deviation between what the user is expected to receive based on the user's TDI versus what the actual amount of insulin delivered to the user is. For every 25% increment of insulin greater than or less than the expected insulin that the user receives, the user's setpoint may be changed by either increasing or decreasing the user's setpoint by 10 mg/dL. In other words, the setpoint is scaled 10mg/dL for each 25% change (over or under the TDI) compared to the user's expected insulin amount.

Specifically, if the total insulin delivery (both automated and manual) in the last 5 hours is significantly greater than the user's typical total daily dose by more than an expected daily variation, the setpoint can be varied as follows in Equation 6:

$$SP_{new}(i) =$$

$$\max\left(\min\left(SP_d + 10\left(\frac{TDI \cdot \frac{5}{24} - \sum_{j=1}^{60} I(i-j)}{0.25 \cdot TDI \cdot \frac{5}{24}}\right), SP_{ub}\right), SP_{lb}\right) \tag{EQ. 6},$$

where $SP_d$ is the default set point, TDI is total daily insulin that is an amount of insulin provided by basal dosages, the 5/24 fraction in the numerator and denominator represents a ratio of hours, in this example 5 hours out of 24 hours, the $SP_{ub}$ is an upper boundary for the set point; the $SP_{lb}$ is a lower boundary for the set point; the value 10, which is a maximum amount of adjustment (either increase/positive or decrease/negative) similar to that in Eq. 5, may be a tunable variable (selectable based on user demographics or preferences), the value 0.25 in the denominator is a tunable value that represents a percentage of the insulin that was delivered over the expected amount of user's delivered insulin (TDI times 5/24). The summation represents the total amount of insulin delivered by both basal amounts and bolus amounts. For example, the summation of $j$ from 1 to 60 (where 60 represents the number of cycles that occurred within the ratio of hours - 60 equals 12 cycles times 5 hours (from the 5/24)) is the deviation between the expected insulin to-be-delivered (represented by I(i)) versus the actual insulin delivered (I(j)) within a 5 hour period of time. The number of hours (5) in the numerator of the hour ratio is linked to the number of cycles (60) in the upper limit of the summation, so when one changes so should the other - if the hours ratio is 4/24 the upper limit of the summation should be 48). In the example, the $SP_{ub}$ is an upper boundary for the setpoint that may be higher than (or lower than) the high target analyte value and the $SP_{lb}$ is a lower boundary for the setpoint that may be lower than (or greater) the low target analyte value.

The factor $TDI \cdot \frac{5}{24} - \sum_{j=1}^{60} I(i-j)$ may be the amount of deviation from the user's expected insulin delivery that is represented by TDI times 5/24. The deviation is divided by 25% (i.e., 0.25) of the user's expected insulin delivery (i.e., 0.25 times TDI times 5/24).

The factor $\frac{TDI \cdot \frac{5}{24} - \sum_{j=1}^{60} I(i-j)}{0.25 \cdot TDI \cdot \frac{5}{24}}$ may be referred to as Y for ease of discussion. In Equation 6, a minimum function (min) takes the minimum of the sum of (the default setpoint $SP_d$ + 10 (Y)) or the setpoint upper boundary $SP_{ub}$, the quantities may be in units of mg/dL. And then a maximum function (max) may take the maximum of the result of the minimum (min) function and a setpoint lower boundary $SP_{lb}$. The output of the maximum function (max) is the new setpoint, $SP_{new}(i)$ for the respective iteration $i$.

In an operational example, the user's default setpoint $SP_d$ may be 120 mg/dL, assume the user's TDI is 24 Units, on average 5/24ths of the TDI should be 5 Units. But assume the actual amount delivered was 6 Units, the deviation would be (-) 1 Units. The difference of 5 Units minus 6 Units is a negative (-)1 Units in the numerator, which is divided by 25% (0.25) of 5 units (or 1.25 Units). The quotient equals (-) 0.8. In this example, the sum of 120 + 10x(-0.8) = 112 mg/dL. The value of 112 mg/dL may be compared to the upper boundary of the setpoint, $SP_{ub}$ (for example, 180 mg/dL) to determine a minimum between the two values using the minimum function. The minimum function returns the minimum value between the two values, 112 mg/dL and $SP_{ub}$. Here, the minimum value is 112 mg/dL. The minimum value (112 mg/dL) is input to a maximum function for comparison to the $SP_{lb}$, such as 70 mg/dL. The output from the maximum function is the maximum between 112 mg/dL and the SPib, 70 mg/dL. Here, the maximum is 112 mg/dL, which, in this specific operational example, is the new setpoint, $SP_{new}(i)$ for the respective iteration i.

A purpose of Equation 6 is to perform real time, or substantially real time, adaptation of a user's setpoint, adjustment or modification of the user's setpoint for approximately every 25% more insulin than the expected hourly TDI that the user receives, Equation 6 reduces the user's setpoint approximately 10 mg/dL. Of course, the percentage over (i.e., represented by 0.25) may range from 5%-10%, 10%-35%, 20%-28% or the like, and the tunable value 10 may also be selected from a range, such as 5 to 25, 8 to 15 or the like. In essence, Equation 6 provides for every 25% greater insulin delivery in the previous 5 hours than would be expected by the user's TDI values, the system would reduce the user's setpoint by 10 mg/dL, and vice versa. The window over which total insulin delivery is evaluated can also be modified, from the current 5 hours (represented as the terms 5/24 and the number of cycles within the summation term, j = 1:60) to longer durations, such as 12 or 24 hours, or shorter durations, such as 2 hours or 1 hour, or the like.

Equation 5 is directing to a first technique for adjusting a user's setpoint based on the monitoring of the user's blood glucose measurement values (for glucose performance) and adjusting based on the monitored blood glucose measurement values, and Equation 6 is directed to a second technique for adjusting a user's setpoint based on monitoring of the insulin delivered to the user based with respect to an expected amount of insulin to be delivered. In a further example, the two techniques for adjusting the user's setpoint may be combined so a user's set point may be adjusted based on glucose performance (i.e., how well the user's blood glucose measurement values stay within range (e.g., 5-20% of the user's setpoint for each cycle, or the like) of the user's setpoint, which may be any of $SP_{new}$ or $SP_{old}$ or $SP_{final}$), and the amount of insulin delivered over a period of time. The equations may be combined as the output of both have the same units, mg/dL.

It is anticipated that any discrepancies between the two techniques of Equations 5 and 6 may be resolved using a weighted average, maximum value, trust index (prioritization based on glucose control outcomes, such as how well the user's glucose concentration blood glucose measurements) remains within the upper and lower setpoints) or other techniques. It is also envisioned that that two techniques may be combined in one equation, as shown in Equation 7 below.

For example,

$$SP_{new}(i) = \max\left(\min\left(SP_d - 10\left(\left(\frac{\max\left(0, \frac{n_{g>180}}{288} - 0.15\right)}{0.05}\right) - \left(\frac{\max\left(0, \frac{n_{g<70}}{288} - 0.015\right)}{0.005}\right)\right.\right.\right.$$
$$\left.\left.\left. + 10\left(\frac{TDI \cdot \frac{5}{24} - \sum_{j=1}^{60} I(i-j)}{0.25 \cdot TDI \cdot \frac{5}{24}}\right)\right), SP_{ub}\right), SP_{lb}\right) \qquad \text{(Eq. 7)},$$

where the values and tunable parameters are the same as those described above with reference to Equation 5 and Equation 6.

When TDI of a user is lacking, a default value may be selected that is clinically reasonable, such as 24 Units or 15-25 Units, or the like, at device initiation. Alternatively, the setpoint adjustment technique of Equation 6 may be implemented after a user has sufficient insulin delivery history (e.g., 48 hours out of the last 54 hours or the like).

In the overnight period, the setpoint may be adjusted to be more conservative because the user is not bolusing themselves, so over the last 6 hours, insulin delivery should be lower than during the daytime, ultimately resulting in a lowered setpoint (SP). During the daytime period, the system may be more aggressive depending upon a selected implementation philosophy. For example, a first philosophy may be, if the user's blood glucose measurement values are persistently high, the user setpoint should be higher because the setpoint adjustment model 180 may have set the user setpoint too low. While an alternative philosophy may be, if the user's blood glucose measurement values are persistently high, the setpoint adjustment model 180 may be set to be more aggressive and lower the setpoint. The setpoint adjustments made by the setpoint adjustment model 180 carefully balanced using the disclosed equations, techniques and processes. Additional care may be implemented by using the following example of an adaptivity scheme.

In a further example in which a full 24 hours of blood glucose measurement data and insulin delivery data are not available, the new setpoint can be implemented as part of an adapted scheme similar to pre-existing adaptivity schemes, as follows in Equation 8:

$$SP_{final} = \left(1 - 0.2 \cdot N_{days}\right) \cdot SP_{old} + 0.2 \cdot N_{days} SP_{new} \qquad \text{(EQ. 8)},$$

where $SP_{final}$ is a final setting that takes in account a preset setpoint value, such as $SP_{old}$, by weighting the $SP_{new}$ is a setpoint from either Equation 5 or 6, but dependent on a determination of the degree of trust in the current data. Of course, other adaptivity schemes may be used. For example, the value 0.2 is a tunable parameter based on how much trust or confidence the algorithm has in the available data. In an example, an $SP_{new}$ value that is derived from only 12 hours of blood glucose measurement values and insulin delivery history may be considered less trustworthy than an $SP_{new}$ value derived from 24 hours of blood glucose measurement values and insulin delivery history, $SP_{old}$ may be a previous iteration of $SP_{new}$ or may be some default value, and $N_{days}$ may be a number of 24 hour days that the data is accumulated over. $SP_{final}$ is a final setting that takes in account a preset setpoint value, such as $SP_{old}$, by weighting the

$SP_{old}$ value greater than the $SP_{new}$ value. In an example, the adaptivity scheme of Eq. 7 or other adaptivity schemes may be applied every 24 hours, but may also, or alternatively, be applied whenever a wearable drug delivery device is replaced, such as every 72-96 hours or the like. Of course, adaptivity equations other than Eq. 8 may be used in the setting of the final setpoint, $SP_{final}$.

Other technical features may be readily apparent to one skilled in the art from the following figures, descriptions, and claims.

Some examples of the disclosed device or processes may be implemented, for example, using a storage medium, a computer-readable medium, or an article of manufacture which may store an instruction or a set of instructions that, if executed by a machine (i.e., processor or controller), may cause the machine to perform a method and/or operation in accordance with examples of the disclosure. Such a machine may include, for example, any suitable processing platform, computing platform, computing device, processing device, computing system, processing system, computer, processor, or the like, and may be implemented using any suitable combination of hardware and/or software. The computer-readable medium or article may include, for example, any suitable type of memory unit, memory, memory article, memory medium, storage device, storage article, storage medium and/or storage unit, for example, memory (including non-transitory memory), removable or non-removable media, erasable or non-erasable media, writeable or re-writeable media, digital or analog media, hard disk, floppy disk, Compact Disk Read Only Memory (CD-ROM), Compact Disk Recordable (CD-R), Compact Disk Rewriteable (CD-RW), optical disk, magnetic media, magneto-optical media, removable memory cards or disks, various types of Digital Versatile Disk (DVD), a tape, a cassette, or the like. The instructions may include any suitable type of code, such as source code, compiled code, interpreted code, executable code, static code, dynamic code, encrypted code, programming code, and the like, implemented using any suitable high-level, low-level, object-oriented, visual, compiled and/or interpreted programming language. The non-transitory computer readable medium embodied programming code may cause a processor when executing the programming code to perform functions, such as those described herein.

Certain examples of the present disclosure were described above. It is, however, expressly noted that the present disclosure is not limited to those examples, but rather the intention is that additions and modifications to what was expressly described herein are also included within the scope of the disclosed examples. Moreover, it is to be understood that the features of the various examples described herein were not mutually exclusive and may exist in various combinations and permutations, even if such combinations or permutations were not made express herein, without departing from the spirit and scope of the disclosed examples. In fact, variations, modifications, and other implementations of what was described herein will occur to those of ordinary skill in the art without departing from the spirit and the scope of the disclosed examples. As such, the disclosed examples are not to be defined only by the preceding illustrative description.

While the examples may have been described with reference to a closed loop algorithmic implementation, variations of the disclosed examples may be implemented to enable open loop use. The open loop implementations allow for use of different modalities of delivery of insulin such as smart pen, syringe or the like. For example, the disclosed AP application and algorithms may be operable to perform various functions related to open loop operations, such as the generation of prompts requesting the input of information such as weight or age. Similarly, a dosage amount of insulin may be received by the AP application or algorithm from a user via a user interface. Other open-loop actions may also be implemented by adjusting user settings or the like in an AP application or algorithm.

Program aspects of the technology may be thought of as "products" or "articles of manufacture" typically in the form of executable code and/or associated data that is carried on or embodied in a type of non-transitory, machine readable medium. Storage type media include any or all of the tangible memory of the computers, processors or the like, or associated modules thereof, such as various semiconductor memories, tape drives, disk drives and the like, which may provide non-transitory storage at any time for the software programming. It is emphasized that the Abstract of the Disclosure is provided to allow a reader to quickly ascertain the nature of the technical disclosure. It is submitted with the understanding that it will not be used to interpret or limit the scope or meaning of the claims. In addition, in the foregoing Detailed Description, various features are grouped together in a single example for streamlining the disclosure. This method of disclosure is not to be interpreted as reflecting an intention that the claimed examples require more features than are expressly recited in each claim. Rather, as the following claims reflect, inventive subject matter lies in less than all features of a single disclosed example. Thus, the following claims are hereby incorporated into the Detailed Description, with each claim standing on its own as a separate example. In the appended claims, the terms "including" and "in which" are used as the plain-English equivalents of the respective terms "comprising" and "wherein," respectively. Moreover, the terms "first," "second," "third," and so forth, are used merely as labels and are not intended to impose numerical requirements on their objects.

The foregoing description of examples has been presented for the purposes of illustration and description. It is not intended to be exhaustive or to limit the present disclosure to the precise forms disclosed. Many modifications and variations are possible in light of this disclosure. It is intended that the scope of the present disclosure be limited not by this detailed description, but rather by the claims appended hereto. Future filed applications claiming priority to this application may claim the disclosed subject matter in a different manner and may generally include any set of one or more limitations as variously disclosed or otherwise demonstrated herein.

While exemplary embodiments have been described herein, it should be appreciated that various changes in form and detail may be made without departing from the intended scope as defined in the appended claims. Although the invention has been explained above and is defined in the enclosed claims, the present invention can - in addition and alternatively - be defined as mentioned in the following embodiments:

1. A computer-implemented method, comprising:

- determining a user is initiating a wearable drug delivery device for an initial use, and in response to determining the initial use:

    setting a high analyte target value and a low analyte target value;
    setting an initial basal amount of a liquid drug;
    starting a set duration and causing the wearable drug device to begin delivery of the initial basal amount;

- obtaining sensor analyte values at predetermined time intervals within the set duration;
- evaluating a respective sensor analyte value against the high target analyte value and the low target analyte value;
- increasing a high target count for each obtained sensor analyte value being greater than the high target analyte value, and increasing a low target count for each obtained sensor analyte value being less than the low target analyte value,
- modifying the initial basal amount at predetermined time increments within the set duration by an adjustment amount of liquid drug;
- wherein for each predetermined time increment, the adjustment amount is determined based on the current high target count and the current low target count.

2. The computer-implemented method of embodiment 1, wherein the adjustment amount is determined based on a set adjustment factor and a variable count factor; and
wherein for each predetermined time increment, the variable count factor is determined based on the current high target count and the current low target count.

3. The computer-implemented method of embodiment 2, wherein the set adjustment factor is a set amount of liquid drug which is:

- selected from 0.005 to 0.4, specifically from 0.01 to 0.2, more specifically from 0.05 to 0.1, and/or
- equal to at least 0.005 units of the liquid drug, specifically at least 0.05 units of the liquid drug.

4. The computer-implemented method of any one of embodiments 2 to 3, wherein the variable count factor is a positive integer which is multiplied with the set adjustment factor to determine the adjustment amount.

5. The computer-implemented method of any one of embodiments 2 to 4, wherein the variable count factor is increased by one integer per each number of high target counts corresponding to the number of predetermined time intervals fitting within the predetermined time increment.

6. The computer-implemented method of any one of embodiments 2 to 5, wherein the variable count factor is always decreased by one integer for each predetermined time increment, and additionally by one integer per each number of low target counts corresponding to the number of predetermined time intervals fitting within the predetermined time increment.

7. The computer-implemented method of any one of the preceding embodiments wherein determining the user is initiating the drug delivery device for the initial use, comprises at least one of:

- receiving an input from a user input device indicating that the user is a new user;
- receiving a signal from a controller indicating that the user is a new user; and/or
- establishing a wireless communication connection with a controller device and receiving a signal indicating the user is a new user.

8. The computer-implemented of any one of the preceding embodiments, further comprising:
selecting the initial basal amount from a default basal amount, wherein the basal amount is a number of units of the liquid drug delivered per a time increment, and the number of units has a range of 0.5 to 4.0 units, specifically a range of 1.0 to 3.0 units and the time increment has a range of 0.2 to 6.0 hours, specifically a range of 0.5 to 3.0 hours.

9. The computer-implemented of any one of the preceding embodiments, wherein the set duration is at least one of:

- a period of time over which a number of the obtained sensor analyte values is greater than a predetermined number of sensor analyte values, and
- a period of time selected from between 24 hours to 72 hours, specifically 36 hours to 48 hours.

10. The computer-implemented of any one of the preceding embodiments, wherein the high analyte target value is a first blood glucose measurement value, and/or the low analyte target value is a second blood glucose measurement value.

11. The computer-implemented of any one of the preceding embodiments, wherein the predetermined time increment is selected based on a number of the predetermined time intervals, specifically, wherein the predetermined time increment is selected from 1 to 60 predetermined time intervals, specifically from 2 to 30 predetermined time intervals, and more specifically from 5 to 15 predetermined time intervals, for instance, 6 predetermined time intervals.

12. The computer-implemented of any one of the preceding embodiments, wherein the predetermined time interval is selected between 1 to 10mins, specifically between 2 to 5mins.

13. The computer-implemented of any one of the preceding embodiments, wherein after the set duration, a drug delivery algorithm is applied based on at least the obtained sensor analyte values and/or the modified initial basal amounts.

14. A wearable drug delivery device, comprising:

    a reservoir that is operable to contain a liquid drug;
    a pump mechanism fluidly coupled to the reservoir;
    a processor; and
    a memory storing instructions that, when executed by the processor, the processor is operable to perform the computer-implemented method of any one of the preceding embodiments.

15. A drug delivery system, comprising:

- a wearable drug delivery device having:

      a reservoir that is operable to contain a liquid drug;
      a pump mechanism fluidly coupled to the reservoir;
      a first processor configured to drive the pump mechanism; and

- a hand-held device comprising a second processor and memory storing instructions that, when executed by the first and/or the second processor, cause the first and/or the second processor to perform the computer-implemented method of any one of the embodiments 1 to 13.

16. The drug delivery system of embodiment 15, further comprising:

- an analyte sensor configured to obtain sensor analyte values.

17. A computer-implemented method for automatic adjustment of a setpoint for a target sensor analyte value comprising:

- obtaining sensor analyte value related data over a period of time;
- comparing the obtained sensor analyte value related data to reference sensor analyte value related data;
- based on the comparison, evaluating if an adjustment of the setpoint is needed;
- based on the result of the evaluation indicating a need for adjustment, performing a modification operation of the previous setpoint for the target sensor analyte value.

18. The computer-implemented method of embodiment 17, wherein the sensor analyte value related data comprises data of sensor analyte values and/or data of drug deliveries.

19. The computer-implemented method of any one of embodiments 17 or 18, wherein comparing is performed at predetermined time intervals within the period of time.

20. The computer-implemented method of any one of embodiments 17 to 19, wherein the sensor analyte value related data comprises data of sensor analyte values, and wherein comparing comprises:

- comparing a respective sensor analyte value to a high target analyte value and to a low target analyte value;
- maintaining a high target count of a number of the respective sensor analyte values being greater than the high target analyte value; and
- maintaining a low target count of a number of the respective sensor analyte values being less than the low target analyte value.

21. The computer-implemented method of embodiment 20, wherein the evaluation comprises:

evaluating the high target count against a high target count threshold;
evaluating the low target count against a low target count threshold.

22. The computer-implemented method of embodiment 21, wherein the high target count threshold is a high target percentage of time threshold, and wherein evaluating the high target count against the high target count threshold further comprises:

- determining relative to the period of time, a percentage of time during which the sensor analyte values are greater than the high target value exceeds the high target percentage.

23. The computer-implemented method of embodiment 22, wherein the modification operation further comprises:

- in response to the determined percentage of time exceeding the high target percentage, decreasing the setpoint for the target sensor analyte value by a preset setpoint value.

24. The computer-implemented method of embodiment 23, wherein decreasing the setpoint for the target sensor analyte value by the preset setpoint value is based on a number of percentage increments that the determined percentage of time is greater than the high target percentage.

25. The computer-implemented method of embodiment 21, wherein the modification operation comprises:

- decreasing the setpoint of the target sensor analyte value by a preset setpoint value for each percentage increment over a percentage of time that the high target count is greater than the high target count threshold.

26. The computer-implemented method of any one of embodiments 21 to 25, wherein the low target count threshold is a low target percentage of time threshold, and wherein evaluating the low target count against the low target count threshold further comprises:

- determining relative to the period of time, a percentage of time during which the sensor analyte values are less than the low target value exceeds the low target percentage.

27. The computer-implemented method of embodiment 26, wherein the modification operation further comprises:

- in response to the determined percentage of time exceeding the low target percentage, increasing the setpoint for the target sensor analyte value by a preset setpoint value.

28. The computer-implemented method of embodiment 27, wherein increasing the setpoint for the target sensor analyte value by the preset setpoint value is based on a number of percentage increments that the determined percentage of time is greater than the low target percentage.

29. The computer-implemented method of any one of embodiments 17 to 28, wherein the sensor analyte value related data comprises data of drug deliveries, and wherein comparing comprises comparing past drug deliveries and expected drug deliveries over a partial period of time, and wherein an adjustment is needed if based on the comparison a deviation between the past drug deliveries and expected drug deliveries is evaluated.

30. The computer-implemented method of embodiment 29, wherein the modification operation further comprises:

- in response to the deviation indicating that the past drug deliveries being less than the expected drug deliveries, increasing the setpoint for the target sensor analyte value by a preset setpoint value, and
- in response to the deviation indicating that the past drug deliveries being greater than the expected drug deliveries, increasing the setpoint for the target sensor analyte value by a preset setpoint value.

31. The computer-implemented method of embodiment 30, wherein adjusting the setpoint for the target sensor analyte value by the preset setpoint value is based on a number of percentage increments that the determined deviation deviates from the expected drug deliveries.

32. The computer-implemented method of any one of embodiments 17 to 31, wherein

- the period of time is selected between 12 hours to 72 hours, specifically between 24 hours to 48 hours, and/or
- if at least dependent on embodiment 30, wherein the partial period of time is selected between 1 hours to 6 hours, specifically between 2 hours to 5 hours.

33. The computer-implemented method of any one of embodiments 17 to 32, wherein after the modification operation, the adjusted setpoint is evaluated against an upper setpoint boundary and/or a setpoint lower boundary, and wherein the adjusted setpoint is only then set as new setpoint if it is within at least one of the upper setpoint boundary and the lower setpoint boundary.

34. A wearable drug delivery device comprising:

a processor; and
a memory storing instructions that, when executed by the processor, configure the processor to perform the computer-implemented method according to any one of embodiments 17 to 33.

35. A drug delivery system, comprising:

- a wearable drug delivery device having:

a reservoir that is operable to contain a liquid drug;
a pump mechanism fluidly coupled to the reservoir;
a first processor configured to drive the pump mechanism; and
- a hand-held device comprising a second processor and memory storing instructions that, when executed by the first and/or the second processor, cause the first and/or the second processor to perform the computer-implemented method of any one of the embodiments 17 to 33.

36. The drug delivery system of embodiment 35, further comprising:

- an analyte sensor configured to obtain sensor analyte values.

While exemplary embodiments have been described herein, it should be appreciated that various changes in form and detail may be made without departing from the intended scope as defined in the appended claims. Although the invention has been explained above and is defined in the enclosed claims, the present invention can - in addition and alternatively - be defined as mentioned in the following embodiments:

1. A method, comprising:

determining a user is initiating a wearable drug delivery device for an initial use;
in response to the initial use, setting a high analyte target value and a low analyte target value;
adjusting a drug delivery algorithm to begin delivery of a basal amount of a liquid drug;

modifying the basal amount by a set amount of liquid drug at predetermined time increments after a set duration; and

after the set duration, readjusting the drug delivery algorithm according to respective sensor analyte values of a number of sensor analyte values stored during the set duration.

2. The method of embodiment 1, wherein determining the user is initiating the drug delivery device for the initial use, further comprises:
receiving an input from a user input device indicating that the user is a new user.

3. The method of any one of the preceding embodiments, wherein determining the user is initiating the drug delivery device for the initial use, further comprises:
receiving a signal from a controller indicating that the user is a new user.

4. The method of any one of the preceding embodiments, wherein determining the user is initiating the drug delivery device for the initial use, further comprises:

establishing a wireless communication connection with a controller device; and
receiving a signal indicating the user is a new user.

5. The method of any one of the preceding embodiments, further comprising:
selecting the basal amount from a default basal amount, wherein the basal amount is a number of units of the liquid drug delivered per a time increment, and the number of units has a range of 1.00 unit to 3.00 units and the time increment has a range from 0.5 hours to 3.00 hours.

6. The method of any one of the preceding embodiments, wherein the modification of the basal amount at the predetermined time increment is based on a sensor analyte value received at a time corresponding to the predetermined time increment, and the sensor analyte value is from the number of sensor analyte values.

7. The method of any one of the preceding embodiments, wherein the set duration is a period of time over which the number of sensor analyte values that are stored is greater than predetermined number of sensor analyte values.

8. The method of any one of the preceding embodiments, wherein the set duration is a period of time selected from: 24 hours, 48 hours or 72 hours.

9. The method of any one of the preceding embodiments, wherein the set amount of the liquid drug indicated by the modification is equal to at least 0.005 units of the liquid drug.

10. The method of any one of the preceding embodiments, wherein the high analyte target value is a first blood glucose measurement value, and the low analyte target value is a second blood glucose measurement value.

11. The method of any one of the preceding embodiments, wherein the set amount of the liquid drug indicated by the modification is either a positive set amount or a negative set amount based on a respective sensor analyte value obtained from the number of sensor analyte values and that corresponds to the predetermined time increment.

12. The method of embodiment 11, wherein the set amount of the liquid drug indicated by the modification is a positive set amount when the respective sensor analyte value corresponds to the predetermined time increment is greater than the high analyte target value.

13. The method of any one of embodiments 11 or 12, wherein the set amount of the liquid drug indicated by the modification is a negative set amount when the respective sensor analyte value corresponds to the predetermined time increment is less than the low analyte target value.

14. A wearable drug delivery device, comprising:

a reservoir that is operable to contain a liquid drug;
a pump mechanism fluidly coupled to the reservoir;
a processor; and
a memory storing instructions that, when executed by the processor, the processor is operable to:

determine a user is initiating the wearable drug delivery device for an initial use;

in response to the initial use, set a high analyte target value and a low analyte target value;

adjust a drug delivery algorithm to begin delivery of basal amount of the liquid drug;

modify the basal amount by a set amount of the liquid drug at predetermined time increments after a set duration; and

after the set duration, readjust the drug delivery algorithm according to respective sensor analyte values of a number of sensor analyte values stored during the set duration.

15. The wearable drug delivery device of embodiment 14, wherein the determining the user is initiating the wearable drug delivery device for the initial use, comprises the processor being operable to:
receive an input from a user input device indicating that the user is a new user.

16. The wearable drug delivery device of any one of embodiments 14 or 15, wherein the determining the user is initiating the wearable drug delivery device for the initial use, comprises the processor being operable to:
receive a signal from a controller indicating that the user is a new user.

17. The wearable drug delivery device of any one of embodiments 14 to 16, wherein the determining the user is initiating the wearable drug delivery device for the initial use, comprises the processor being operable to:

establish a wireless communication connection with a controller device; and
receive a signal indicating the user is a new user.

18. The wearable drug delivery device of any one of embodiments 14 to 17, wherein the modification of the basal amount at the predetermined time increment is based on a sensor analyte value received at a time corresponding to the predetermined time increment, and the sensor analyte value is one of the number of sensor analyte values.

19. The wearable drug delivery device of any one of embodiments 14 to 18, wherein the set duration is a period of time over which the number of sensor analyte values that are stored is greater than a predetermined number of sensor analyte values.

20. The wearable drug delivery device of any one of embodiments 14 to 19, wherein the set duration is a period of time selected from: 24 hours, 48 hours or 72 hours.

21. The wearable drug delivery device of any one of embodiments 14 to 20, wherein the set amount of the liquid drug indicated by the modification is equal to at least 0.005 units of the liquid drug.

22. The wearable drug delivery device of any one of embodiments 14 to 21, wherein the high analyte target value is a first blood glucose measurement value, and the low analyte target value is a second blood glucose measurement value.

23. The wearable drug delivery device of any one of embodiments 14 to 22, wherein the set amount of the liquid drug indicated by the modification is either a positive set amount or a negative set amount based on a respective sensor analyte value obtained from the number of sensor analyte values and that corresponds to the predetermined time increment.

24. The wearable drug delivery device of embodiment 23, wherein the set amount of the liquid drug indicated by the modification is a positive set amount when the respective sensor analyte value corresponds to the predetermined time increment is greater than the high analyte target value.

25. The wearable drug delivery device of any one of embodiments 23 or 24, wherein the set amount of the liquid drug indicated by the modification is a negative set amount when the respective sensor analyte value corresponds to the predetermined time increment is less than the low analyte target value.

26. A method, comprising:

receiving a sensor analyte values over a period of time;
at time of receipt of a respective sensor analyte value, comparing the respective sensor analyte value to a high target analyte value and to a low target analyte value;

maintaining a high target count of a number of the respective sensor analyte values are greater than the high target analyte value;

maintaining a low target count of a number of the respective sensor analyte values are less than the low target analyte value;

evaluating the high target count against a high target count threshold;

evaluating the low target count against a low target count threshold; and

based on a result of either the evaluation of the high target count or the evaluation of the low target count indicating a need for a setpoint adjustment, performing a modification operation of a sensor analyte value setpoint.

27. The method of embodiment 26, wherein evaluating the high target count against the high target count threshold, further comprises:

determining a percentage of time that the sensor analyte values are greater than the high target count threshold exceeded a high target percentage.

28. The method of any one of embodiments 26 or 27, wherein the modification operation further comprises:

in response to the determined percentage of time exceeding the high target percentage, decreasing a sensor analyte value setpoint for a target sensor analyte value by a preset setpoint value.

29. The method of embodiment 28, wherein decreasing the setpoint for the target sensor analyte value by the preset setpoint value is based on a number of percentage increments that the determined percentage of time is greater than the high target percentage.

30. The method of embodiment 26, wherein the modification operation of the sensor analyte value setpoint comprises:

decreasing the sensor analyte value setpoint by a preset setpoint value for each percentage increment over a percentage of time that the high target count is greater than the high target count threshold.

31. The method of embodiment 26, wherein evaluating the low target count against the low target count threshold, further comprises:

determining a percentage of time that the sensor analyte values are less than the low target count threshold.

32. The method of embodiment 31, wherein the modification operation further comprises:

in response to the determined percentage of time being less than the low target percentage, increasing the setpoint for a target sensor analyte value by a preset setpoint value.

33. The method of embodiment 32, wherein increasing the setpoint for the target sensor analyte value by the preset setpoint value is based on a number of percentage increments that the determined percentage of time is less than the low target percentage.

34. The method of embodiment 26, wherein the period of time is one of: 12 hours, 24 hours, 36 hours, or 48 hours.

35. A wearable drug delivery device comprising:

a processor; and
a memory storing instructions that, when executed by the processor, configure the processor to:

receive a sensor analyte values over a period of time;

at time of receipt of a respective sensor analyte value, compare the respective sensor analyte value to a high target analyte value and to a low target analyte value;

maintain a high target count of a number of the respective sensor analyte values are greater than the high target analyte value;

maintain a low target count of a number of the respective sensor analyte values are less than the low target analyte value;

evaluate the high target count against a high target count threshold;

evaluate the low target count against a low target count threshold; and

based on a result of either the evaluation of the high target count or the evaluation of the low target count indicate need for a setpoint adjustment, perform a modification operation of a sensor analyte value setpoint.

36. The wearable drug delivery device of embodiment 35, wherein when the processor is evaluating the high target count against the high target count threshold, further comprises:
determine a percentage of time that the sensor analyte values are greater than the high target count threshold exceeded a high target percentage.

37. The wearable drug delivery device of embodiment 36, wherein when the processor performs the modification operation, the processor is further operable to:
in response to the determined percentage of time exceeding the high target percentage, decreasing a setpoint for a target sensor analyte value by a preset setpoint value.

38. The wearable drug delivery device of embodiment 37, wherein when the processor is decreasing the setpoint for the target sensor analyte value by the preset setpoint value, the processor is operable to base the decrease on a number of percentage increments the determined percentage of time is greater than the high target percentage.

39. The wearable drug delivery device of embodiment 35, wherein when the processor performs the modification operation of the sensor analyte value setpoint, the processor is operable to:
decrease the sensor analyte value setpoint by a preset setpoint value for each percentage increment over a percentage of time that the high target count is greater than the high target count threshold.

40. The wearable drug delivery device of embodiment 35, wherein evaluating the low target count against the low target count threshold, the processor is further operable to:
determine a percentage of time that the sensor analyte values are less than the low target count threshold.

41. The wearable drug delivery device of embodiment 40, wherein when performing the modification operation, the processor is further operable to:
in response to the determined percentage of time being less than the low target percentage, increase the setpoint for a target sensor analyte value by a preset setpoint value.

42. The wearable drug delivery device of embodiment 41, wherein, the processor is further operable, when increasing the setpoint for the target sensor analyte value by the preset setpoint value, to base the increase on a number of percentage increments the determined percentage of time is less than the low target percentage.

43. The wearable drug delivery device of embodiment 35, wherein the processor is operable to select the period of time from a time of: 12 hours, 24 hours, 36 hours, or 48 hours.

**Claims**

1. A computer-implemented method, comprising:

   - determining a user is initiating a wearable drug delivery device for an initial use, and in response to determining the initial use:

      setting a high analyte target value and a low analyte target value;
      setting an initial basal amount of a liquid drug;
      starting a set duration and causing the wearable drug device to begin delivery of the initial basal amount;

      - obtaining sensor analyte values at predetermined time intervals within the set duration;
      - evaluating a respective sensor analyte value against the high target analyte value and the low target analyte value;
      - increasing a high target count for each obtained sensor analyte value being greater than the high target analyte value, and increasing a low target count for each obtained sensor analyte value being less than the low target analyte value,
      - modifying the initial basal amount at predetermined time increments within the set duration by an adjustment amount of liquid drug;
      wherein for each predetermined time increment, the adjustment amount is determined based on the current high target count and the current low target count.

2. The computer-implemented method of claim 1, wherein the adjustment amount is determined based on a set adjustment factor and a variable count factor; and

wherein for each predetermined time increment, the variable count factor is determined based on the current high target count and the current low target count.

3. The computer-implemented method of claim 2, wherein the set adjustment factor is a set amount of liquid drug which is:

   - selected from 0.005 to 0.4, specifically from 0.01 to 0.2, more specifically from 0.05 to 0.1, and/or
   - equal to at least 0.005 units of the liquid drug, specifically at least 0.05 units of the liquid drug.

4. The computer-implemented method of any one of claims 2 to 3, wherein the variable count factor is a positive integer which is multiplied with the set adjustment factor to determine the adjustment amount.

5. The computer-implemented method of any one of claims 2 to 4, wherein the variable count factor is increased by one integer per each number of high target counts corresponding to the number of predetermined time intervals fitting within the predetermined time increment.

6. The computer-implemented method of any one of claims 2 to 5, wherein the variable count factor is always decreased by one integer for each predetermined time increment, and additionally by one integer per each number of low target counts corresponding to the number of predetermined time intervals fitting within the predetermined time increment.

7. The computer-implemented method of any one of the preceding claims wherein determining the user is initiating the drug delivery device for the initial use, comprises at least one of:

   - receiving an input from a user input device indicating that the user is a new user;
   - receiving a signal from a controller indicating that the user is a new user; and/or
   - establishing a wireless communication connection with a controller device and receiving a signal indicating the user is a new user.

8. The computer-implemented of any one of the preceding claims, further comprising:
selecting the initial basal amount from a default basal amount, wherein the basal amount is a number of units of the liquid drug delivered per a time increment, and the number of units has a range of 0.5 to 4.0 units, specifically a range of 1.0 to 3.0 units and the time increment has a range of 0.2 to 6.0 hours, specifically a range of 0.5 to 3.0 hours.

9. The computer-implemented of any one of the preceding claims, wherein the set duration is at least one of:

   - a period of time over which a number of the obtained sensor analyte values is greater than a predetermined number of sensor analyte values, and
   - a period of time selected from between 24 hours to 72 hours, specifically 36 hours to 48 hours.

10. The computer-implemented of any one of the preceding claims, wherein the high analyte target value is a first blood glucose measurement value, and/or the low analyte target value is a second blood glucose measurement value.

11. The computer-implemented of any one of the preceding claims, wherein the predetermined time increment is selected based on a number of the predetermined time intervals, specifically, wherein the predetermined time increment is selected from 1 to 60 predetermined time intervals, specifically from 2 to 30 predetermined time intervals, and more specifically from 5 to 15 predetermined time intervals, for instance, 6 predetermined time intervals.

12. The computer-implemented of any one of the preceding claims, wherein the predetermined time interval is selected between 1 to 10mins, specifically between 2 to 5mins.

13. The computer-implemented of any one of the preceding claims, wherein after the set duration, a drug delivery algorithm is applied based on at least the obtained sensor analyte values and/or the modified initial basal amounts.

14. A wearable drug delivery device, comprising:

   a reservoir that is operable to contain a liquid drug;
   a pump mechanism fluidly coupled to the reservoir;

a processor; and
a memory storing instructions that, when executed by the processor, the processor is operable to perform the computer-implemented method of any one of the preceding claims.

**15.** A drug delivery system, comprising:

- a wearable drug delivery device having:

a reservoir that is operable to contain a liquid drug;
a pump mechanism fluidly coupled to the reservoir;
a first processor configured to drive the pump mechanism; and

- a hand-held device comprising a second processor and memory storing instructions that, when executed by the first and/or the second processor, cause the first and/or the second processor to perform the computer-implemented method of any one of the claims 1 to 13.

**16.** The drug delivery system of claim 15, further comprising:

- an analyte sensor configured to obtain sensor analyte values.

**17.** A computer-implemented method for automatic adjustment of a setpoint for a target sensor analyte value comprising:

- obtaining sensor analyte value related data over a period of time;
- comparing the obtained sensor analyte value related data to reference sensor analyte value related data;
- based on the comparison, evaluating if an adjustment of the setpoint is needed;
- based on the result of the evaluation indicating a need for adjustment, performing a modification operation of the previous setpoint for the target sensor analyte value.

**18.** The computer-implemented method of claim 17, wherein the sensor analyte value related data comprises data of sensor analyte values and/or data of drug deliveries.

**FIG. 1**

200

DETERMINE A USER IS INITIATING A WEARABLE DRUG DELIVERY DEVICE FOR AN INITIAL USE 202

↓

IN RESPONSE TO THE INITIAL USE, SET A HIGH ANALYTE TARGET VALUE AND A LOW ANALYTE TARGET VALUE 204

↓

ADJUST A DRUG DELIVERY ALGORITHM TO BEGIN DELIVERY OF A BASAL AMOUNT OF A LIQUID DRUG 206

↓

MODIFY THE BASAL AMOUNT BY A SET AMOUNT OF LIQUID DRUG AT PREDETERMINED TIME INCREMENTS WITHIN A SET DURATION 208

↓

AT THE END OF THE SET DURATION, READJUST THE DRUG DELIVERY ALGORITHM ACCORDING TO RESPECTIVE SENSOR ANALYTE VALUES OF A NUMBER OF SENSOR ANALYTE VALUES STORED DURING THE SET DURATION 210

**FIG. 2**

300

RECEIVE A SENSOR ANALYTE VALUES OVER A PERIOD OF TIME 302

↓

AT TIME OF RECEIPT OF A RESPECTIVE SENSOR ANALYTE VALUE, COMPARE THE RESPECTIVE SENSOR ANALYTE VALUE TO A HIGH TARGET ANALYTE VALUE AND TO A LOW TARGET ANALYTE VALUE 304

↓

MAINTAIN A HIGH TARGET COUNT OF A NUMBER OF THE RESPECTIVE SENSOR ANALYTE VALUES ARE GREATER THAN THE HIGH TARGET ANALYTE VALUE 306

↓

MAINTAIN A LOW TARGET COUNT OF A NUMBER OF THE RESPECTIVE SENSOR ANALYTE VALUES ARE LESS THAN THE LOW TARGET ANALYTE VALUE 308

↓

EVALUATE THE HIGH TARGET COUNT AGAINST A HIGH TARGET COUNT THRESHOLD 310

↓

EVALUATE THE LOW TARGET COUNT AGAINST A LOW TARGET COUNT THRESHOLD 312

↓

BASED ON A RESULT OF EITHER THE EVALUATION OF THE HIGH TARGET COUNT OR THE EVALUATION OF THE LOW TARGET COUNT INDICATING A NEED FOR A SETPOINT ADJUSTMENT, PERFORMING A MODIFICATION OPERATION OF A SENSOR ANALYTE VALUE SETPOINT 314

**FIG. 3**

Europäisches
Patentamt

European
Patent Office

Office européen
des brevets

**EUROPEAN SEARCH REPORT**

Application Number

EP 23 16 4522

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2022/023391 A1 (MICHELICH ALAN JOHN [US] ET AL) 27 January 2022 (2022-01-27) | 1-4,7-18 | INV. G16H20/17 |
| A | * [0029], [0036], [0097], [0114], [0274], [0281], [0446]-[0447] * | 5,6 | |
| Y | Anonymous: "Fine-Tuning Your Basal Insulin Regimen", , 29 March 2021 (2021-03-29), pages 1-6, XP093058437, Retrieved from the Internet: URL:https://web.archive.org/web/2021032900 4652/https://www.diabetesselfmanagement.co m/managing-diabetes/treatment-approaches/g etting-down-to-basals/ [retrieved on 2023-06-27] | 1-4,7-18 | |
| A | * page 5 par 3 * | 5,6 | |
| A | Anonymous: "Insulin dosing", , 8 November 2020 (2020-11-08), pages 1-35, XP093058350, Retrieved from the Internet: URL:https://web.archive.org/web/2020110813 4259/https://www.straighthealthcare.com/in sulin-dosing.html [retrieved on 2023-06-27] * page 7, bullet point 5 * | 1-18 | TECHNICAL FIELDS SEARCHED (IPC) G16H |

The present search report has been drawn up for all claims

| Place of search | Date of completion of the search | Examiner |
|---|---|---|
| Munich | 16 August 2023 | Bankwitz, Robert |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

**EP 23 16 4522**

This annex lists the patent family members relating to the patent documents cited in the above-mentioned European search report.
The members are as contained in the European Patent Office EDP file on
The European Patent Office is in no way liable for these particulars which are merely given for the purpose of information.

16-08-2023

| Patent document cited in search report | | Publication date | Patent family member(s) | | Publication date |
|---|---|---|---|---|---|
| US 2022023391 | A1 | 27-01-2022 | CN | 112313755 A | 02-02-2021 |
| | | | EP | 3815106 A1 | 05-05-2021 |
| | | | JP | 2022514804 A | 16-02-2022 |
| | | | US | 2022023391 A1 | 27-01-2022 |
| | | | WO | 2020002428 A1 | 02-01-2020 |

EPO FORM P0459

For more details about this annex : see Official Journal of the European Patent Office, No. 12/82